# EUROPEAN PATENT SPECIFICATION

(11) **EP 3 565 906 B1**
(45) Date of publication and mention of the grant of the patent: **27.01.2021**
(21) Application number: 17832949.6
(22) Date of filing: 19.12.2017
(51) Int. Cl.: C12Q 1/6851, C12Q 1/6862, C12Q 1/6827

(54) **QUANTIFYING DNA SEQUENCES**
QUANTIFIZIERUNG VON DNA-SEQUENZEN
QUANTIFICATION DE SÉQUENCES D'ADN

(30) Priority: 05.01.2017 SE 1750003
(43) Date of publication of application: 13.11.2019
(73) Proprietor: Tervisetehnoloogiate Arenduskeskus AS, 50410 Tartu (EE)
(72) Inventor: KRJUTSKOV, Kaarel, 50416 Tartu (EE); KOEL, Mariann, 50416 Tartu (EE); KERE, Juha, 113 62 Stockholm (SE); SALUMETS, Andres, 51005 Tartu (EE)
(74) Representative: Barker Brettell Sweden AB
(86) International application number: PCT/EP2017/083613
(87) International publication number: WO 2018/127408

(56) References cited:
- WO-A1-2013/009175
- WO-A1-2013/106807
- WO-A1-2013/192292
- WO-A1-2014/089536
- US-A1- 2016 068 907
- OLGA KONDRASHOVA ET AL: "High-Throughput Amplicon-Based Copy Number Detection of 11 Genes in Formalin-Fixed Paraffin-Embedded Ovarian Tumour Samples by MLPA-Seq", PLOS ONE, vol. 10, no. 11, 16 November 2015 (2015-11-16), page e0143006, XP055459930, DOI: 10.1371/journal.pone.0143006

## Description

### TECHNICAL FIELD

The present embodiments generally relate to methods and kits for quantifying target DNA sequences, and in particular to such methods and kits that can be used for genetic screening and molecular diagnostic purposes.

### BACKGROUND

Nucleic acid and its variances detection through amplification is a leading technique to determine DNA mutations, genotype Single Nucleotide Polymorphisms (SNPs), and analyze DNA Copy-Number Variances (CNVs). DNA variances are analyzed directly from DNA strands using only DNA to DNA manipulation principles to convert a chemical signal, i.e., a nucleotide in the DNA strand, to an electronic signal impulse, i.e., a nucleotide presented on the screen.

RNA analysis, e.g., gene expression analysis, is technically performed through *in vitro* synthesized complementary DNA (cDNA) that provides stable template for laboratory manipulations and signal detection. Once cDNA is synthesized, it is amplified for the analysis.

Those DNA and RNA sequences and CNVs are generally denoted as nucleic acid Sequence Of Interest (SOI).

Amplification is generally needed to multiply original nucleic acid molecules and ensure that the original strands are converted to detection compatible molecules that are not lost during processing. This process is typically denoted library preparation in the meaning of Next Generation Sequencing (NGS) or just allele amplification in more straightforward methods, where variances of nucleic acid strands are visualized on different gel systems or analyzed by strand mass and/or fluorescence differences.

Thermostable DNA polymerases and specific primer sequences, in proper reaction conditions, are used to amplify the SOI. Primers are commonly designed downstream and upstream of the analyzed position to ensure optimum priming conditions and proper length of amplicons. The SOI is incorporated into clone molecules during PCR. Later, the pool of amplicons is sequenced by NGS or analyzed by different assays. The advantage of polymerase-mediated SOI conversion into amplicons is hypothesis-free design where all possible variances between specific primers are involved in clonal amplification. Main shortages Thermostable Ligation (TL) mediated amplification and detection is a simple and sensitive technique that permits the user to detect any previously defined SOI molecules. TL mediated reaction provides the basis for simple "+/-" assays with the power to distinguish DNA sequences that differ by a single base as disclosed in [1, 2]. The TL reaction has advantages over PCR with respect to specificity, sensitivity, and simplicity.

Multiplex Ligation-dependent Probe Amplification (MLPA®) technique uses minimum two MLPA® oligonucleotides to discriminate SOI sequence variance [3] or copy-number difference [4]. Once both MLPA® oligonucleotides are hybridized to their SOI during stringent overnight hybridization, the MLPA® oligonucleotides can be ligated to form a complete MLPA® probe. A pair of PCR primers is used to amplify the MLPA® probes, which are later identified by capillary electrophoresis.

Another assay, named as Digital ANalysis of Selected Regions (DANSR®), enables detection of extensive genome scale copy number differences. More specifically, DANSR® is designed for whole chromosome aneuploidies in Non-Invasive Prenatal Testing (NIPT) [5].

A recently published technique [9], TempO-Seq®, performs SOI analysis based on target RNA and complementary DNA oligonucleotide hybridization and RNA T4 ligase mediated analysis. Two DNA oligonucleotides define the studied locus and the analysis is performed by NGS.

Methods for detecting nucleic acid sequences are disclosed in [10-12]. In [10], target nucleic acid sequences may be highly abundant in a sample, and attenuator oligonucleotides are needed in order to reduce the number of detection products. In [11], nucleases are provided to selectively degrade unused or excess detectors to enable sensitive detection of target nucleic acid sequences. In [12], presence of a polymorphic site is detected using two competing ligation oligonucleotides.

Two main short-comings are present with the prior art assay techniques. The absolute counting of individual original RNA or DNA molecules is not possible due to the fact that SOI amplification is unbalanced and therefore later detection is biased. Another type of limitation is introduced when only an aliquot of ligated product is carried to amplification step. This may cause lower sequencing covering and allelic drop-out.

US 2016/0068907 discloses ligation assays for detecting and profiling expression products at transcriptome scale.

Thus, there is a need for improved techniques for quantifying DNA sequences that solve shortcomings of the prior art assay techniques.

### SUMMARY

It is a general objective to provide an improved quantification of target DNA sequences.

This and other objectives are met by embodiments as disclosed herein.

An aspect of the embodiments relates to a method for quantifying target DNA sequences. The method comprises contacting, in each container of N separate containers, target DNA sequences, under hybridization conditions, with *M_{L}* left ligation oligonucleotides and *M_{R}* right ligation oligonucleotides. N, *M_{L}* and *M_{R}* are each an integer equal to or larger than two. The *M_{L}* left ligation oligonucleotides comprise, from a 5' end to a 3' end, a sequencing read-1 primer site, a respective first unique molecular identifier (UMI), and a respective sequence complementary to a first segment of a target DNA sequence. The *M_{R}* right ligation oligonucleotides comprise, from a 5' end to a 3' end, a respective sequence complementary to a second segment of the target DNA sequence, a respective second UMI, and a sequencing read-2 primer site. The method also comprises adding, in each container of the N separate containers, a ligating agent capable of ligating together the 3' end of a left ligation oligonucleotide and the 5' end of a right ligation oligonucleotide while hybridized to a target DNA sequence to form a ligated product comprising two UMIs and hybridized to the target DNA sequence. The method further comprises immobilizing, in each container of the N separate containers, the ligated product in complex with the target DNA sequence onto a solid phase having preference for DNA sequences of a length in terms of number of deoxyribonucleotides equal to or larger than a minimum length, and removing a supernatant. The method additionally comprises amplifying, in each container of the N separate containers, the ligated product in presence of a left amplification primer and a right amplification primer to form an amplified product comprising two UMIs and one barcode sequence. The left amplification primer comprises, from a 5' end to a 3' end, a first common sequence and the sequencing read-1 primer site. The right amplification primer comprises, from a 5' end to a 3' end, a second common sequence, a barcode sequence and a ligation oligonucleotide binding site complementary to the sequencing read-2 primer site. The method also comprises mixing together the content of the N separate containers. The method further comprises sequencing at least a respective portion of the amplified products by addition of a sequencing read-1 primer comprising the sequencing read-1 primer site and a sequencing read-2 primer comprising the read-2 primer site to form respective sequence reads. Each respective sequence reads comprises at least nucleotide sequences of two UMIs, one barcode sequence and a target DNA sequence. The method additionally comprises demultiplexing the sequence reads based on nucleotide sequences of the barcode sequences and mapping the demultiplexed sequence reads to known DNA sequences based on nucleotide sequences of the target DNA sequence. The method further comprises quantifying unique target DNA sequences in the N containers based on the demultiplexed and mapped sequence reads and based on nucleotide sequences of the UMIs.

Another aspect of the embodiments relates to a kit for quantifying target DNA sequences. The kit comprises *M_{L}* left ligation oligonucleotides comprising, from a 5' end to a 3' end, a sequencing read-1 primer site, a respective first UMI and a respective sequence complementary to a respective first segment of a target DNA sequence. The kit also comprises *M_{R}* right ligation oligonucleotides comprising, from a 5' end to a 3' end, a respective sequence complementary to a respective second segment of a target DNA sequence, a respective second UMI and a sequencing read-2 primer site. *M_{L}* and *M_{R}* are each an integer equal to or larger than two. The respective first UMI is different for each of the *M_{L}* left ligation oligonucleotides and the respective second UMI is different for each of the *M_{R}* right ligation oligonucleotides. The kit further comprises a left amplification primer comprising, from a 5' end to a 3' end, a first common sequence and the sequencing read-1 primer site and a right amplification primer comprising, from a 5' end to a 3' end, a second common sequence, a barcode sequence and a ligation oligonucleotide binding site complementary to the sequencing read-2 primer site. The kit additionally comprises a sequencing read-1 primer comprising the sequencing read-1 primer site and a sequencing read-2 primer comprising the read-2 primer site.

The present embodiments provide a quantification of DNA sequences that minimizes PCR-induced bias and enables absolute molecule and allele counting. The present embodiments enable transfer of all ligated products from ligation step to the amplification. A following analysis by sequencing ensures maximum possible coverage of unique DNA molecules at each studied locus.

### BRIEF DESCRIPTION OF THE DRAWINGS

The embodiments, together with further objects and advantages thereof, may best be understood by making reference to the following description taken together with the accompanying drawings, in which:
Figs. 1A-1E schematically illustrate steps, oligonucleotides and primers used in a method for quantifying target DNA sequences according to an embodiment;
Fig. 2 schematically illustrates an amplified double-stranded product according to an embodiment;
Fig. 3 schematically illustrates an amplified product according to an embodiment and the binding of a sequencing read-1 primer and sequencing read-2 primer during a sequencing step according to an embodiment;
Fig. 4 is a flow chart illustrating a method for quantifying target DNA sequences according to an embodiment;
Fig. 5 is a flow chart illustrating additional, optional step of the method shown in Fig. 4;
Fig. 6 is a flow chart illustrating an embodiment of the performing step in Fig. 5;
Fig. 7 schematically illustrates sequencing read-1 and read-2 primer binding sites as together with exact nucleotide length information of sequenced portions according to an embodiment;
Fig. 8 schematically illustrates the principle of molecular manipulation steps through entire laboratory procedure according to an embodiment;
Fig. 9 illustrates the principles of handling multiple samples in a method for quantifying target DNA sequences according to an embodiment;
Fig. 10 illustrates sheared genomic DNAs mimicking cell-free DNA before hybridization;
Fig. 11 illustrates the PCR products after concentration by silica column;
Fig. 12 illustrates the PCR products after bead-based size selection;
Fig. 13 illustrates the PCR products after 16 and 18 cycles of PCR;
Fig. 14 illustrates amplified products ready for sequencing;

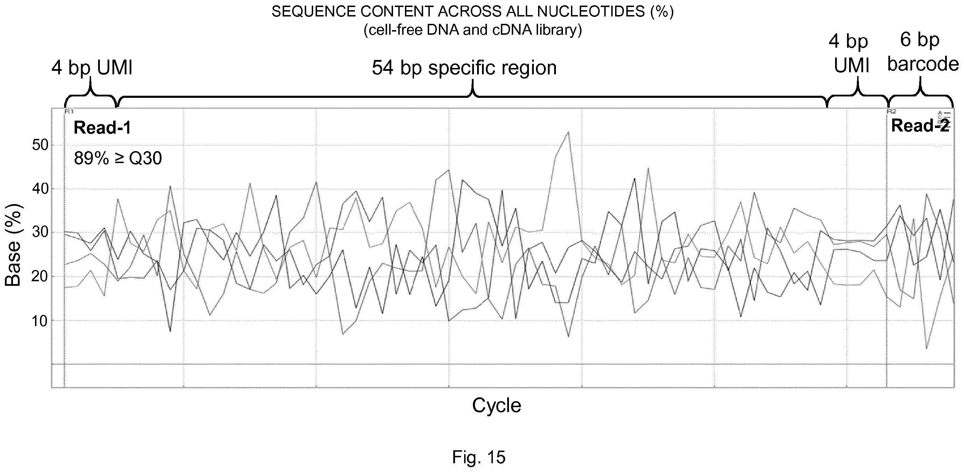

Fig. 15 illustrates sequence content distribution across all four bases after sequencing read-1 and read-2 read;
Fig. 16 is a flow chart illustrating additional, optional steps of the method shown in Fig. 4 during data analysis following sequencing;
Fig. 17 demonstrates the method capture efficiency and reproducibility;
Fig. 18 shows RNA analysis method comparison between "golden standard" RNA-sequencing and method described in an embodiment;
Fig. 19 confirms analyzed samples different clustering based on principal component analysis according to method as described in an embodiment;
Fig. 20 illustrates the results of cell-free DNA analysis over two different chromosomes in diploid DNA and in case of aneuploidy; and
Fig. 21 graphically demonstrates the cell-free DNA based trisomy detection threshold in case of aneuploidy.

### DETAILED DESCRIPTION

Throughout the drawings, the same reference numbers are used for similar or corresponding elements.

The present embodiments generally relate to methods and kits for quantifying target DNA sequences, also referred to as SOIs, and in particular to such methods and kits that can be used for genetic screening and molecular diagnostic purposes.

The quantification of the embodiments enables quantification of target DNA sequences with high specificity and sensitivity even in complex biological samples comprising a plurality of nucleotide sequences. Thus, unspecific amplification and quantification of non-target nucleotide sequences are eliminated or at least significantly reduced.

The present embodiments enable absolute molecule and allele counting. The embodiments also enable pooling together separated amplified products from different patients or samples for any following processing, such as sequencings, thereby significantly simplifying the handling of the amplified products.

Furthermore, the present embodiments allow amplification and later analysis through sequencing all target DNA molecules present in a biological sample to achieve correct DNA sequence quantification even in such samples where the target DNA sequence concentration is relatively low.

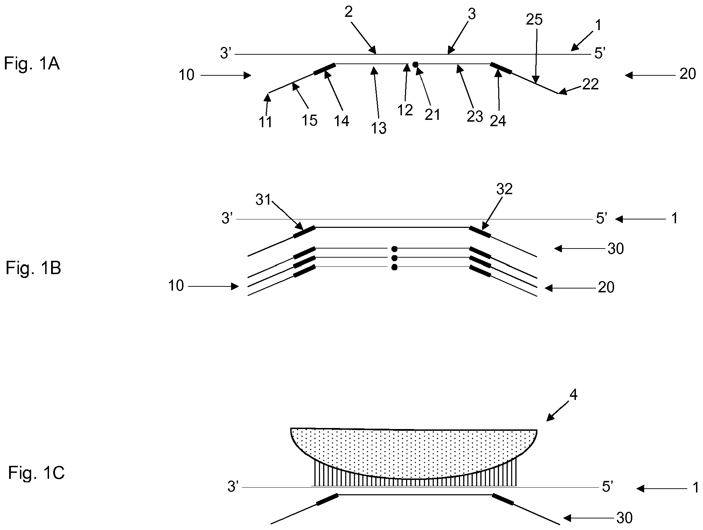

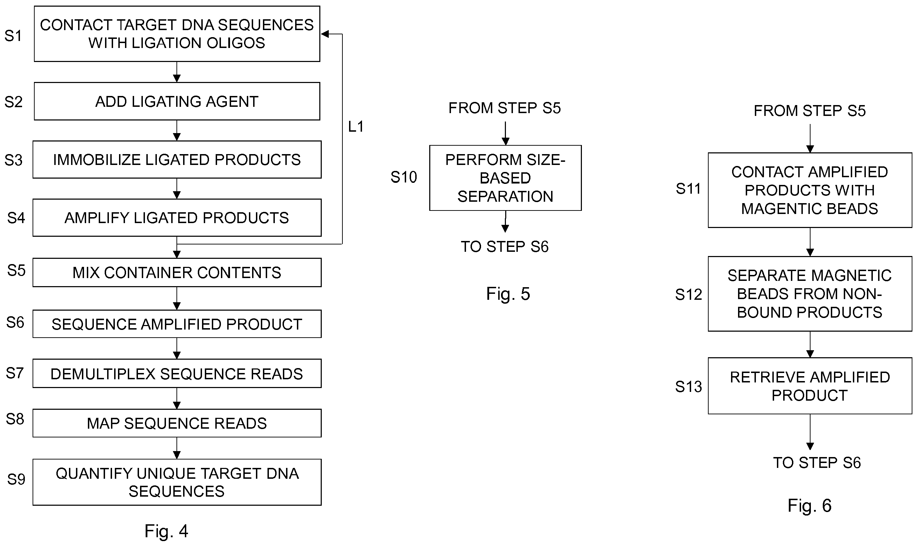

Fig. 4 illustrates a flow chart of a method for quantifying target DNA sequences, also denoted SOI herein, according to an embodiment. Reference is also made to Figs. 1A-1E schematically illustrating steps, oligonucleotides and primers used in the method for quantifying target DNA sequences according to an embodiment. The method comprises contacting, in step S1 and in each container of *N* separate containers, target nucleotide sequences 1, under hybridization conditions, with *M_{L}* left ligation oligonucleotides 10, also denoted first ligation oligonucleotides herein, and *M_{R}* right ligation oligonucleotides 20, also denoted second ligation oligonucleotides herein, see Fig. 1A. *N, M_{L}* and *M_{R}* are each an integer equal to or larger than two.

The *M_{L}* left ligation oligonucleotides 10 comprise, from a 5' end 11 to a 3' end 12, a sequencing read-1 primer site 15, a respective first unique molecular identifier (UMI) 14 and a respective sequence 13 complementary to a respective first segment 2 of a target DNA sequence 1. The *M_{R}* right ligation oligonucleotides 20 comprise, from a 5' end 21 to a 3' end 22, a respective sequence 23 complementary to a respective second segment 3 of a target DNA sequence 1, a respective second UMI 24 and a sequencing read-2 primer site 25.

The method also comprises adding, in step S2 and in each container of the N separate containers, a ligating agent capable of ligating together the 3' end 12 of a left ligation oligonucleotide 10 and the 5' end 21 of a right ligation oligonucleotide 20 while hybridized to a target DNA sequence 1 to form a ligated product 30 comprising two UMIs 30, 31 and hybridized to the target DNA sequence 1, see Fig. 1B.

The method further comprises immobilizing, in step S3 and in each container of the *N* separate containers, the ligated product 30 in complex with the target DNA sequence 1 onto a solid phase 4, see Fig. 1C. The solid phase 4 has preference for DNA sequences of a length in terms of number of deoxyribonucleotides equal to or larger than a minimum length. This step S3 also comprises removing a supernatant.

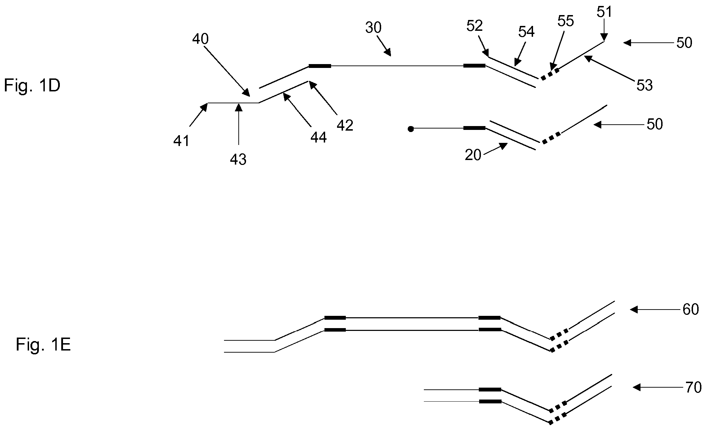

The method additionally comprises amplifying, in step S4 and in each container of the *N* separate containers, the ligated product 30 in presence of a left amplification primer 40 and a right amplification primer 50 to form an amplified product 60, see Figs. 1D and 1E. The left amplification primer 40 comprises, from a 5' end 41 to a 3' end 42, a first common sequence 43 and the complete sequencing read-1 primer site 44. The right amplification primer 50 comprises, from a 5' end 51 to a 3' end 52, a second common sequence 53, a barcode sequence 55, and a ligation oligonucleotide binding site 54 complementary to the sequencing read-2 primer site 25.

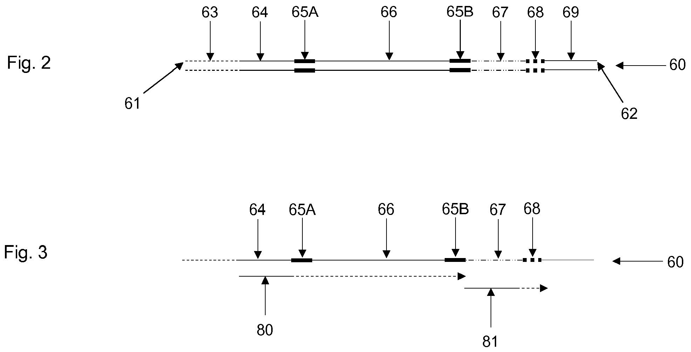

Figs. 1E and 2 illustrate the amplified product 60 resulting from the amplification step S4 shown in Fig. 4. The amplified product 60 comprises, from a 5' end 61 to a 3' end 62, the first common sequence 63, the sequencing read-1 primer site 64, the first UMI 65A, the first segment of the target DNA sequence and the second segment of the target DNA sequence, i.e., the SOI 66, the second UMI 65B, the ligation oligonucleotide binding site 67, the barcode sequence 68 and the second common sequence 69.

The method as shown in Fig. 4 also comprises mixing together the content of the N separate containers in step S5. The method further comprises sequencing, in step S6, at least a respective portion of the amplified product 60 by addition of a sequencing read-1 primer 80, see Fig. 3, comprising the sequencing read-1 primer site 15 and a sequencing read-2 primer 81 comprising the read-2 primer site 25 to form respective sequence reads comprising at least nucleotide sequences of two UMIs 65A, 65B, one barcode sequence 68 and a target DNA sequence 66. The method additionally comprises demultiplexing, in step S7, the sequence reads based on nucleotide sequences of the barcode sequences 68 and mapping, in step S8, the demultiplexed sequence reads to known DNA sequences based on nucleotide sequences of the target DNA sequence 66. The method also comprises quantifying, in step S9, unique target DNA sequences 1 in the *N* containers based on the demultiplexed and mapped sequence reads and based on nucleotide sequences of the UMIs.

The method as shown in Fig. 4 performs an amplification by ligation to achieve a high level of specificity, i.e., amplifying the target DNA sequences 1 while suppressing amplification of any other nucleotide sequences that might be present in a sample in addition to the target DNA sequences 1.

This amplification by ligation is achieved by hybridizing left and right ligation oligonucleotides 10, 20 to a target DNA sequence 1. Thus, the target DNA sequences 1 are contacted with the ligation oligonucleotides 10, 20 under hybridization conditions, during which the sequence 13 complementary to the first segment 2 of a target DNA sequence 1 hybridizes to this first segment 2 and the sequence 23 complementary to the second segment 3 of a target DNA sequence 1 hybridizes to the second segment 3.

Hybridization or hybridization condition denotes the process in which single-stranded nucleotide sequences anneal to complementary nucleotide sequences. Such annealing between complementary nucleotide sequences is dependent on several parameters including, for instance, ionic strength, temperature, length of the oligonucleotides 10, 20, or rather the complementary sequences 13, 23 thereof, and G-C-nucleotides content of the complementary sequences 13, 23.

Complementary as user herein refers both to complete complementarity of nucleotide sequences, in some cases referred to as an identical sequence, as well as complementarity sufficient to achieve the desired binding of nucleotide sequences. Complementary refers to the standard base pairing rules between G-C, A-T and A-U. Certain nucleotides not commonly found in natural nucleotide sequences or chemically synthesized may be included in the nucleotide sequences described herein. Complementarity need not be perfect. In clear contrast, stable duplexes may contain mismatched base pairs, degenerative, or unmatched nucleotides.

The left and right ligation oligonucleotides 10, 20 hybridize to a target DNA sequence 1 as shown in Fig. 1A through the binding between the complementary sequences: the first segment 2 and the sequence 13 complementary to the first segment 2, and the second segment 3 and the sequence 23 complementary to the second segment 3.

The target DNA sequence 1 could be any DNA molecule comprising or consisting of a SOI or a sequence to be quantified. The target DNA sequence 1 may, thus, be a single-stranded or double-stranded DNA sequence, a cell-free DNA sequence, a genomic DNA sequence, a cDNA sequence, or indeed any other DNA sequence from any source. The target DNA sequence 1 is preferably present in a sample, such as biological sample, from a subject, such as an animal subject, preferably a mammal subject, and more preferably a human subject. In such a case, the biological sample could be body fluid sample, such as a blood sample, a blood plasma sample, a saliva sample, a cerebrospinal fluid sample, or an endometrial fluid sample, or a body tissue sample, such as a biopsy sample.

The first segment 2 and the second segment 3 in the target DNA sequence 1 are preferably neighboring or adjacent sequences. This means that the 3' end 12 of a left ligation oligonucleotide 10 and the 5' end 21 of a right ligation oligonucleotide 20 are at adjacent or nearly adjacent positions when the left and right ligation oligonucleotides 10, 20 are hybridized to the target DNA sequence 1. These spatially adjacent positions of the 3' end 12 and the 5' end 21 facilitate ligation of the two ends 12, 21 in step S2 and following addition of the ligating agent.

The ligating agent added in step S2 is capable of ligating together the 3' end 12 of a left ligation oligonucleotide 10 and the 5' end 21 of a right ligation oligonucleotide 20 while hybridized to a target DNA sequence 1 to form a ligated product 30.

The ligated agent thereby covalently joints the ends 12, 21 of the ligation oligonucleotides 10, 20. As is schematically illustrated in Fig. 1B, such ligation of the ends 12, 21 only takes place for ligated oligonucleotides 10, 20 positioned with the ends 12, 21 adjacent each other. This occurs when left and right ligation oligonucleotides 10, 20 are hybridized to a target DNA sequence 1. As is shown in the figure, non-binding ligation oligonucleotides 10, 20 that are not hybridized to a target DNA sequence 1 will consequently not be covalently joint by the ligation agent or are ligated but a comparatively much lower amount or level as compared to ligation oligonucleotides 10, 20 hybridized to a target DNA sequence 1.

The ligated products 30 from step S2, each comprises two UMIs 31, 32, and are each hybridized to a target DNA sequence 1. The ligated products 30, while hybridized to the target DNA sequences 1, are hybridized onto solid phase 4 in step S3. This solid phase 4 has preference for DNA sequences of sufficient lengths. Hence, the solid phase 4 preferably binds to and is thereby capable of immobilizing DNA sequences having a length equal to or larger than a minimum length. Length of a DNA sequence as used herein relates to the number of deoxyribonucleotides of the DNA sequence. Having preference for DNA sequences of certain length furthermore imply that the solid phase 4 binds better to DNA sequences equal to or larger than the minimum length as compared to comparatively shorter DNA sequences. Hence, the solid phase 4 has higher specificity and higher binding strength for DNA sequences of a length equal to or larger than the minimum length as compared to DNA sequences of shorter length.

For instance, the solid phase 4 has preference for ligated products 30 hybridized to a target DNA sequence 1 over ligation oligonucleotides 10, 20 that are not ligated together in step S2. Thus, free ligation oligonucleotides 10, 20 not hybridized to any target DNA sequence 1 and thereby not ligated together to form a ligated product 30 bind with lower binding strength and less to the solid phase 4 and are thereby mainly present in the supernatant.

The immobilization of the ligated product 30 hybridized to the target DNA sequence 1 in step S3 enables removal of the supernatant. This removal of the supernatant implies that free ligation oligonucleotides 10, 20 are removed to thereby mainly keep the ligated product 30 in the N containers and remove non-ligated ligation oligonucleotides 10, 20 by removing the supernatant.

In an embodiment, the solid phase 4 is magnetic beads 4, preferably carboxylated magnetic beads 4, having preference for DNA sequences of a length in terms of number of nucleotides or base pairs equal to or larger than 100 base pairs or nucleotides.

Amplification of the ligated product 30 in step S4 takes place by means of the amplification primers 40, 50 as shown in Fig. 1D. This amplification produces the amplified product 60, typically in two steps. In a first step, the right amplification primer 50 hybridizes to the ligated product 30 produced in step S2. In more detail, the ligation oligonucleotide binding site 54 that is complementary to the sequencing read-2 primer site 25 binds to the sequencing read-2 primer site 25 portion of the ligated product 30. The right amplification primer 50 thereby functions as an amplification primer with the ligated product 30 serving as template for amplification. In this first step, the right amplification primer 50 is extended with the ligated product 30 serving as template for amplification to form an extension product. In a second step, the left amplification primer 40 hybridizes to the extension product produced in the first step. In more detail, the sequencing read-1 prime site 44 hybridizes to a complementary sequence in the extension product. This means that the left amplification primer 40 functions as an amplification primer with the extension product as template for amplification. The result of the second step is the amplified product 60 and its complementary sequence as shown in the upper portion of Fig. 1E.

The amplification in step S4 of Fig. 4 thereby, in an embodiment, comprises two amplification sub-steps, which result in a double stranded amplified product 60. Each such amplification sub-step thereby comprises template-based primer extension using the right amplification primer 50 as amplification primer and the ligated product 30 as template in the first sub-step and the left amplification primer 40 as amplification primer and the extension product from the first sub-step as template in the second sub-step.

The amplified product 60 resulting from the amplification in step S4 comprises two UMIs 65A, 65B and one barcode sequence 68.

The first and second UMIs 14, 24 in the ligation oligonucleotides 10, 20 are preferably specific for each ligation oligonucleotide 10, 20. Thus, it means that the respective first UMIs 14 of the left ligation oligonucleotides 10 added in step S1 preferably all have different nucleotide sequences. For instance, if the first UMI 14 is a random n₁n₂n₃...nₖ sequence, wherein nᵢ, i=1...k, is one of A, T, C and G, then it is possible to have 4^{k} left ligation oligonucleotides 10. Correspondingly, the second UMIs 24 of the right ligation oligonucleotides 20 added in step S1 preferably have different nucleotide sequences. For instance, if the second UMI 24 is a random nm₂n₃...nₖ sequence, wherein nᵢ, i=1...k, is one of A, T, C and G, then it is possible to have 4^{k} right ligation oligonucleotides 20. In total, ligation products 30 comprising the two UMIs 31, 32 preferably have unique combinations of the first and second UMIs 31, 32. Accordingly, it is possible to obtain 4^{2k} unique combinations of UMIs 31, 32.

The first and second UMIs 14, 24 serve to reduce the quantitative bias introduced by replication, i.e., amplification in step S4.

In an embodiment, the first and second UMI 14, 24 is each a random nm₂n₃...nₖ sequence, wherein nᵢ, i=1...k, is one of A, T, C and G. In an embodiment, the parameter k is from 2 up to 6, preferably from 3 up to 5, such as 4.

The barcode sequences 68 introduced into the amplified product 60 by the right amplification primers 50 are preferably sample-specific barcode sequences 68. Hence, in an embodiment, all right amplification primers 50 added to a container of the N containers in step S4 have the same DNA sequence. However, the barcode sequence 55 of right amplification primers 50 added to one of the containers in step S4 preferably differ from the barcode sequence 55 of the right amplification primers 50 added to another container of the N containers.

In an embodiment, the barcode sequence 55 of the right amplification primers 50 and the barcode sequence 68 in the amplified products 60 are preferably sample specific and has a length of 4 up to 16 nucleotides, preferably 4 up to 10 nucleotides, more preferably 6 up to 8 nucleotides, such as 6 nucleotides.

Each amplified product 60 thereby preferably has a combination of the first and second UMIs 65A, 65B and one samples-specific barcode sequence 68.

The ligating agent added in step S2 of Fig. 4 can be any ligating agent capable of ligating together the 3' end 12 of a left ligation oligonucleotide 10 and the 5' end 21 of a right ligation oligonucleotide while hybridized to a target DNA sequence 1.

In an embodiment, the ligating agent is a ligase, and in particular a DNA ligase. A DNA ligase is an enzyme that facilitates the joining of DNA strands together by catalyzing the formation of a phosphodiester bond. Any ligase or DNA ligase capable of ligating together the ends 12, 21 of the ligation oligonucleotides 10, 20 while hybridized to the target nucleotide sequence 1 can be used according to the embodiments.

In a particular embodiment, the ligating agent is a thermostable ligating agent, preferably a thermostable ligase, and in particular a thermostable DNA ligase. For instance, the thermostable ligase could be selected from the illustrative group comprising Ampligase® DNA ligase, Taq DNA ligase, and 9°N™ DNA ligase.

Other non-limiting, but illustrative, examples of DNA ligases include *Escherichia coli* DNA ligase encoded by the *lig* gene; T4 DNA ligase from bacteriophage T4; DNA ligase I, II, III or IV.

In such a case, step S2 of Fig. 4 preferably comprises adding a ligase, preferably a DNA ligase, and more preferably a thermostable ligase or a thermostable DNA ligase, to ligate together the 3' end 12 of a left ligation oligonucleotide 10 and the 5' end 21 of a right ligation oligonucleotide 20 while hybridized to a target DNA sequence 1 to form a ligated product 30.

In an embodiment, the 5' end 21 of the right ligation oligonucleotides 20 comprises a 5' phosphate group, which is indicated in Figs. 1A-1B by the black dot.

In an embodiment, step S4 of Fig. 4 comprises performing 2 to 30 cycles of amplification of the ligated product 30 in presence of the left amplification primer 40 and the right amplification primer 50 to form the amplified product 60. In a preferred embodiment, step S4 comprises performing 10 to 20 cycles of amplification.

In a particular embodiment, the amplification is performed using Polymerase Chain Reaction (PCR) in step S4. In such a case, the amplification preferably comprises performing 2 to 30 cycles, preferably 10 to 20 cycles of PCR.

In the amplification step S4, the right amplification primer 50 may also hybridize to any free right ligation oligonucleotides 20 as indicated in the lower part of Fig. 1D. In more detail, the ligation oligonucleotide binding site 54 in the right amplification primer 50 may hybridize to the sequencing read-2 primer binding site 25 in the right ligation oligonucleotide 20. As a result of this hybridization, the amplification step S4 may produce not only the desired amplified product 60 but also a comparatively shorter side-product 70 as indicated in the lower part of Fig. 1E. This side-product 70, however, has a comparatively much shorter length, in terms of number of nucleotides, as compared to the amplified product 60. Accordingly, the amplified product 60 can easily be separated from the side-product 70, such as in a size-based separation. The side-product 70 does not contain both common sequences, which are required for sequencing. Accordingly, even if the side-product 70 is present in small concentrations it will not disturb sequencing analysis.

Furthermore, the immobilization of the ligated products 30 in step S3 using a solid phase 4 having a preference for DNA sequences longer than the length of the left and right ligation oligonucleotides 10, 20 reduces the amount of right ligation oligonucleotides 20 when starting the amplification in step S4.

In an embodiment, the method comprises an additional, optional step S10 as shown in Fig. 5. This step S10 comprises performing size-based separation of the amplified product 60 from any side-product 70 produced in the amplification in step S4.

Any size-based separation available in the art and capable of separating double-stranded DNA sequences based on lengths of the DNA sequences can be used. Non-limiting, but illustrative, examples of such size-based separations include electrophoresis, such as gel electrophoreses, e.g., by means of agarose or polyacrylamide gels, or capillary electrophoreses; nucleotide sequence separation by silica adsorption, such as using silica membranes, silica columns, or silica beads, such as magnetic silica coated beads; or nucleotide sequence separation by carboxyl adsorption, such as using carboxyl membranes, carboxyl columns or carboxyl beads, such as carboxyl coated magnetic beads.

Fig. 6 is a flow chart illustrating an embodiment of performing the size-based separation. In this embodiment, the method comprises contacting, in step S11, the amplified product 60 with magnetic beads configured to bind single-stranded or double-stranded nucleotide sequences of a target length interval. In such a case, a length of the amplified product 60 falls within the target length interval. The method also comprises separating the magnetic beads from non-bound nucleotide sequences 70 and retrieving, in step S13, the amplified product 60 from the magnetic beads.

In an optional embodiment, the amplified product 60 is concentrated prior to contacting the amplified product 60 with the magnetic beads in step S11. In such a case, the method comprises step S10, which comprises concentrating the amplified product 60 prior to performing the size-based separation.

The concentration step S10 may, for instance, involve silica column based concentration to reduce the volume of the pooled or mixed content from the N separate containers. Other techniques known in the art to concentrate double-stranded nucleotide sequences could alternatively be used.

The concentration step S10 may have the additional advantage of purifying the amplified product 60. Thus, some side-products 70 may be removed in the concentration step S10, in particular if using a silica column for concentration of the amplified products 60.

The method of the embodiments can advantageously be used in connection with detection of single nucleotide mutations and polymorphism. In such a case, different left ligation oligonucleotides 10, preferably differing from each other in one nucleotide at the 3' end 12, and/or different right ligation oligonucleotides 20, preferably differing from each other in one nucleotide at the 5' end 21, could be used.

In these embodiments, *M_{L}* and *M_{R}* are each an integer equal or larger than two. In a particular embodiment, *M_{L}* and *M_{R}* are each four. In such a case, a first ligation oligonucleotide of the four ligation oligonucleotides comprises G as the respective nucleotide, a second ligation oligonucleotide of the four ligation oligonucleotides comprises C as the respective nucleotide, a third ligation oligonucleotide of the four ligation oligonucleotides comprises A as the respective nucleotide, and a fourth ligation oligonucleotide of the four ligation oligonucleotides comprises T as the respective nucleotide.

In these embodiments, the ligation oligonucleotide of the *M_{L}* and *M_{R}* ligation oligonucleotides having a respective oligonucleotide that is complementary to the corresponding interrogated nucleotide in the first segment 2 or the second segment 3 of the target DNA sequence 1 will hybridize to the target DNA sequence 1 at a higher binding affinity than the other ligation oligonucleotides for which the respective oligonucleotide is not complementary to the interrogated nucleotide in the target nucleotide sequence 1. Accordingly, a high binding specificity is achieved even if the portion of ligation oligonucleotides hybridizing to the first or second segment 2, 3 of the target DNA sequence 1 only differs from each other at a single nucleotide.

In an embodiment, two nucleotides of the target DNA sequence 1 can be interrogated, one with the respective nucleotide at the 3' end 12 of the *M_{L}* left ligation oligonucleotides 10 and one with the respective nucleotide at the 5' end 21 of the *M_{R}* right ligation oligonucleotides 20. The two interrogated nucleotides in the target DNA sequence 1 are preferably adjacent nucleotides, i.e., positioned next to each other in the target DNA sequence 1.

The *M_{L}* left ligation oligonucleotides 10 preferably have the same sequencing read-1 primer site 15 and optionally also the same sequence 13 complementary to the first segment 2 of the target DNA sequence 1 except the nucleotide at the 3' end 12. The *M_{R}* right ligation oligonucleotides 20 preferably have the same sequencing read-2 primer site 25 and optionally also the same sequence 23 complementary to the second segment 3 of the target DNA sequence 1 except the nucleotide at the 5' end 21. Thus, in an embodiment, the differences between the multiple left or right ligation oligonucleotides 10, 20 are, preferably, in the interrogated nucleotide and the UMIs 14, 24.

The method for quantifying target DNA sequence 1 can advantageously be used to process multiple target DNA sequences 1 in parallel by running steps S1 and S4 in the N separate containers as schematically illustrated by the line L1 in Fig. 4. The resulting amplified products 60 from these parallel amplifications can then be pooled together, i.e., mixed in step S5. This is possible due to the introduction of the barcode sequence 55 with the right amplification primers 50. In such a case, a respective barcode sequence 68 is introduced into each amplified product 60. The barcode sequences 68 can thereby be used to identify the source, i.e., patient sample, from which the respective amplified product 60 originates.

The amplified products 60 following the mixing in step S5 are sequenced in step S6 of Fig. 4. The sequencing is achieved by means of a sequencing read-1 primer 80 and a sequencing read-2 primer 81. The sequencing read-1 primer 80 comprises the sequencing read-1 primer site 15 and the sequencing read-2 primer 81 comprises the read-2 primer site 25. The result of the sequencing in step S6 is respective sequence reads comprising at least nucleotide sequences of two UMIs 65A, 65B, one barcode sequence 68 and a target DNA sequence 66.

Thus, step S6 comprises sequencing at least a portion of an amplified product 60 comprising the UMIs 65A, 65B, a sequence 66 corresponding to the first segment 2 and the second segment 3 of the target DNA sequence 1 and the barcode sequence 68.

In a particular embodiment, step S6 comprises *in situ* sequencing the at least a portion of the amplified product 60 immobilized onto a solid support based on the first common sequence 63 and/or the second common sequence 69.

In such an embodiment, the solid support preferably comprises immobilized nucleotide sequences complementary to the first common sequence 63 and/or immobilized nucleotide sequences complementary to the second common sequence 65.

The *in situ* sequencing of step S6 preferably comprises *in situ* sequencing by synthesis of the at least a portion of the amplified product 60.

For instance, the first common sequence 63 is one of the P5 sequence (5'-AATGATACGGCGACCACCGA-3', SEQ ID NO: 1) and the P7 sequence (5'-CAAGCAGAAGACGGCATACGAGAT-3', SEQ ID NO: 2). The second common sequence 69 is then the other of the P5 sequence and the P7 sequence. In such an example, the ILLUMINA® sequencing technology could be used to *in situ* sequence at least a portion of the amplified product 60 by synthesis. In more detail, the amplified product 60 is immobilized on a flow cell surface designed to present the amplified sequence 60 in a manner that facilitates access to enzymes while ensuring high stability of surface bound amplified product 60 and low non-specific binding of fluorescently labeled nucleotides.

Sequence By Synthesis (SBS) uses two or four fluorescently labeled nucleotides to sequence the amplified products 60 on the flow cell surface in parallel. During each sequencing cycle, a single labeled deoxynucleoside triphosphate (dNTP) is added to the nucleic acid chain. The nucleotide label serves as a terminator for polymerization so after each dNTP incorporation, the fluorescent dye is imaged to identify the base and then enzymatically cleaved to allow incorporation of the next nucleotide. More information of the ILLUMINA® sequencing technology can be found in [6],

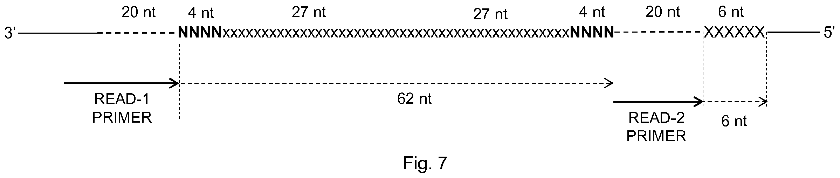

By performing an *in situ* sequencing, such as using ILLUMINA® sequencing technology, the two sequence reads obtained for each immobilized amplified product 60 can be related to each other, i.e., assigning the two sequence reads to the same amplified product 60. The first sequence read is obtained using the sequencing read-1 primer 80 and preferably generates the nucleotide sequence of the first and second UMIs and the target DNA sequence, see Fig. 7. Correspondingly, the second sequence read is obtained using the sequencing read-2 primer 81 and preferably generates the nucleotide sequence of the barcode sequence. The first sequence read and the second sequence read of the same amplified product 60 are thereby connected to each other through the spatial site of the immobilized amplified product 60.

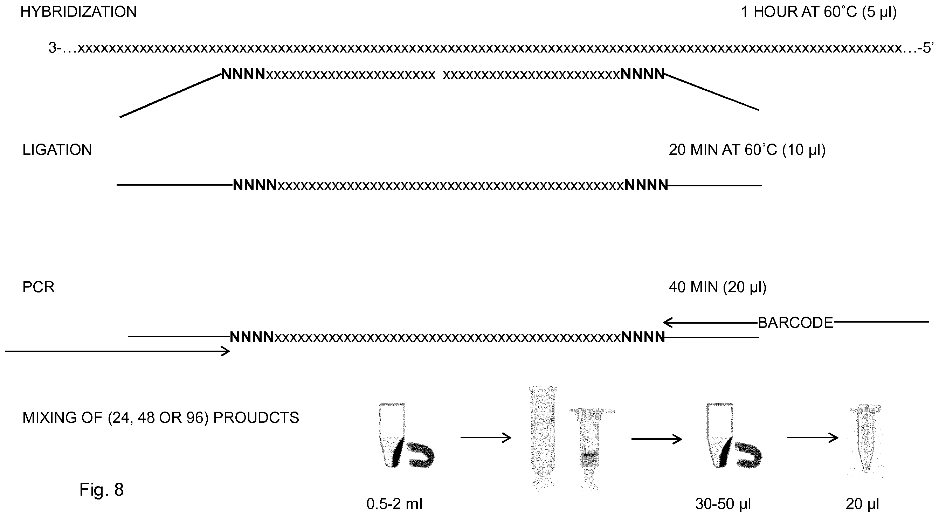

Fig. 8 schematically illustrates steps, oligonucleotides and primers used in an embodiment. The upper sequence is a target DNA sequence. The upper part of the figure also illustrates a left ligation oligonucleotide with a first UMI indicated as four nucleotides Ns and a right ligation oligonucleotide with a second UMI indicated as four nucleotides Ns. The ligation oligonucleotides are allowed to hybridize to the target DNA sequence, such as for at least one hour, for instance, at 60°C.

An advantage of the embodiment is that a small volume is needed, e.g., equal to or less than 5 µl as schematically indicated in the Fig. 8. This is preferred in applications where the starting sample comprising the target DNA sequence is only available in a small volume.

A DNA ligase, such as the thermostable Taq DNA ligase, is added and the ligation reaction takes place at, for instance, about 20 minutes at 60°C to form a ligated product shown in the middle part of Fig. 8.

Thereafter a PCR-based amplification reaction is performed as indicated in Fig. 8 with the addition of the amplification primers, of which the right amplification primer contains the barcode sequence. The PCR-based amplification is typically run in, for instance, about 30 to 50 minutes, such as 40 minutes, depending on the number of amplification cycles and the used thermostable (hot-start) DNA polymerase. The output of the PCR-based amplification is the amplified product present in a volume of, for instance, about 20 µl.

The above three steps, i.e., hybridization, ligation and PCR-based amplification, are performed in separate containers for different samples. The content of these containers can then be mixed as indicated in the lower part of Fig. 8. The mixed content is then preferably concentrated, such as using a silica column, to achieve concentrated amplified products, such as going from about 0.5-2 ml down to about 30-50 µl. The figure also indicates a size-based separation using, for instance, carboxylated magnetic beads to form the desired double-stranded amplified products in a small volume of, for instance, about 20 µl.

Please note that each sample, i.e., container of the N containers, may comprise zero, one or multiple, i.e., at least two, different target DNA sequences in any number of copies to be quantified by the embodiments.

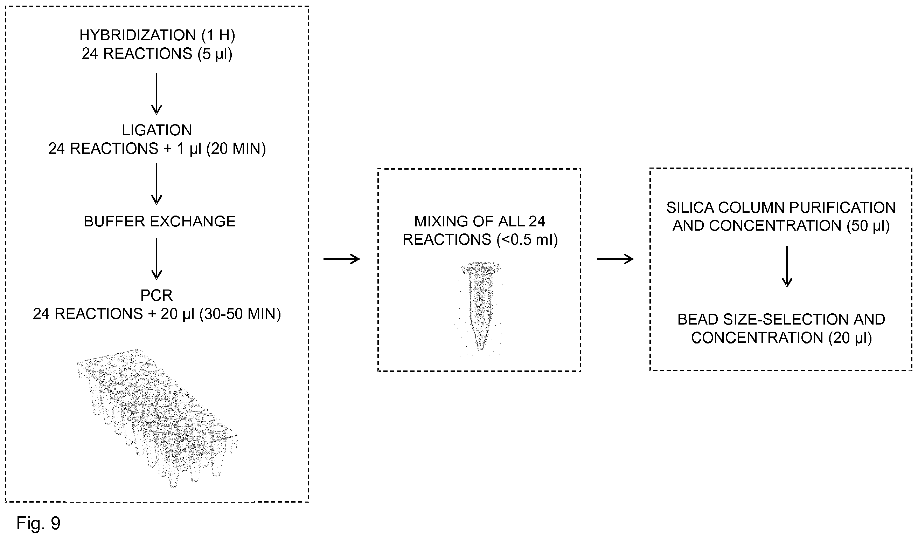

Fig. 9 illustrates the principles of handling multiple samples in an embodiment. The left part of the figure illustrates the method steps, i.e., hybridization, ligation and PCR-based amplification that are performed in separate containers. These separate containers could be, for instance, individual reaction tubes or a plate with multiple wells or reaction tubes, such as 24, 48 or 96 wells or reaction tubes, e.g., a 24/48/96-well PCR-type plate.

The figure also indicates mixing all reaction products together in a container, such as reaction tube, followed by silica column purification and concentration, and carboxylated bead-based size selection and concentration.

As mentioned in the foregoing, following the hybridization, ligation, immobilization and amplification steps, which are conducted in separate containers for each sample or patient, the contents from the amplification steps are mixed and pooled together. However, the present embodiments use patient- or sample-specific barcode sequences. Accordingly, the sequence reads obtained in step S6 can be demultiplexed, i.e., separated, in step S7 based on the respective barcode sequence in sequenced amplified products.

In an embodiment, step S7 comprises dividing the sequence reads into groups having a same nucleotide sequence of the barcode sequence with at most n mismatches allowed for nucleotide sequences of barcode sequences in the same group. In an embodiment, n is zero or one.

Thus, the sequence reads are preferably divided into different groups based on the nucleotide sequences of the barcode sequences so that sequence reads having the same barcode sequence are included in the same group. In this grouping or division of sequence reads, it is possible to accept up to one nucleotide mismatch between barcode sequences that belong to the same group.

This grouping of sequence reads in step S7 is mainly performed by the respective sequence 2-reads as these contain the nucleotide sequences of the barcode sequences. However, by the association and connection between sequence 1-reads and sequence 2-reads due to the spatial immobilization of amplified products during the sequencing step S6 the division of sequence 2-reads also means that the associated sequence 1-reads are also grouped into these different groups.

In an embodiment, step S8 comprises dividing the demultiplexed sequence reads into groups having a same nucleotide sequence of the target DNA sequence with at most m mismatches allowed for nucleotide sequences of target DNA sequences in a same group. In an embodiment, m is an integer equal to or larger than 0 but no larger than 10, preferably no larger than 7, such as no larger than 6 or 5.

Thus, within each group of sequence reads following the demultiplexing in step S7, the sequence reads are further divided into groups, or sub-groups, based on the nucleotide sequences of the target DNA sequences. This means that sequence reads having the same target DNA sequence are grouped into the same group. In this grouping or division of sequence reads, it is possible to accept up to 10 nucleotide mismatches, preferably up to 5-6 nucleotide mismatches, between target DNA sequences that belong to the same group. Mismatches are allowed in order to accept possible polymerase-caused nucleotide substitutions during library preparation and sequencing procedure.

In an embodiment, only sequence reads, in which the sequence 1-read has a full size, i.e., number of nucleotides for the first and second UMIs and the intermediate target DNA sequence are processed in step S8. Fig. 7 illustrates this concept, in which the full size is 62 nucleotides. Hence, in such an example, only sequence reads, in which the sequence 1-read has the full length of 62 nucleotides (nt) are processed in step S8, whereas shorter sequence 1-reads are discarded and not included in the further processing.

In the above presented example, the full length 62 nt sequence 1-read comprises 8 nt of UMIs and 54 nt of the SOI, i.e., the target DNA sequence. The 54 nt SOI is mapped using known target DNA sequences as reference by allowing up to 10, preferably up to 5-6 mismatches over the 54 nt. This mapping thereby divides the sequence reads to have the same nucleotide sequences allowing the above-mentioned mismatches.

The above-mentioned n and m mismatches are preferably set to clearly distinguish sequence reads from each other but still be flexible enough to allow sequencing introduced nucleotide biases by the employed polymerase.

In an embodiment, step S9 of Fig. 4 comprises quantifying unique target DNA sequences based on determining a number of target DNA sequences in the N containers having a nucleotide sequence with at most m mismatches, having a nucleotide sequence of the barcode sequence with at most n mismatches and having different combinations of the first UMI and the second UMI.

Identical previously mapped sequence reads with identical UMIs are merged as amplification duplicates and are depicted as one unique sequence read. Thus, sequence reads within the same group, i.e., the same barcode sequence with at most n mismatches and the same target DNA sequence with at most m mismatches, are further analyzed based on UMI sequences. Thus, sequence reads having the same UMI sequence are merged as PCR amplification duplicates and not counted as multiple unique sequence reads but rather as one unique sequence read. The quantification in step S9 thereby quantifies the number of DNA molecules having a same target DNA sequence in a given sample. This is possible by only counting sequence reads with unique combinations of the first and second UMIs to thereby remove identical PCR clones obtained during the amplification in step S4.

In some embodiments, a UMI threshold can be used in the quantification in step S9. This means that the same UMI should be detected at least two or more times during sequencing to be sure that some polymerase step has not caused "new unique UMI" from some already amplified UMI due to nucleotide change. The UMI threshold is recommended in the case of deep sequencing depths. The UMI threshold then defines the minimum number of times that the same UMI, i.e., the same combination of first and second UMIs, needs to be detected in order to be counted.

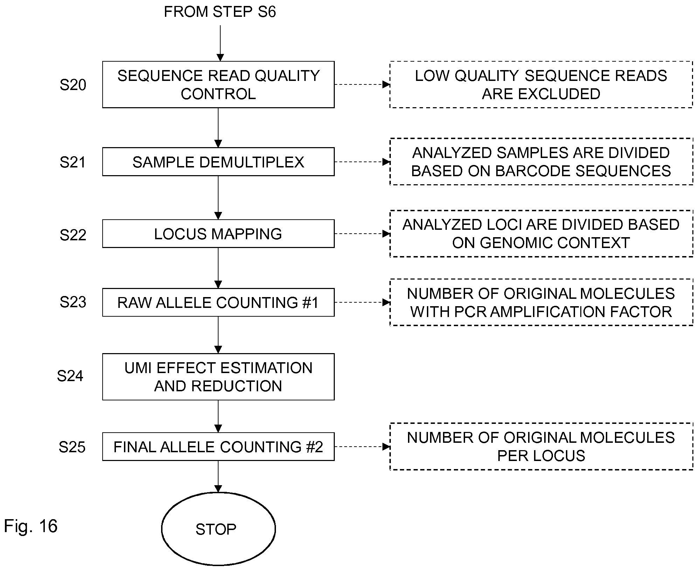

Fig. 16 is a flow chart illustrating an embodiment of steps S7 to S9 in Fig. 4. These additional steps relate to the data processing of the sequencing data as obtained in step S6 in Fig. 4. A first step S20 comprises performing a sequence read quality control, in which sequence reads of low quality are excluded. Generally, sequencing of a nucleotide sequence also generates quality information of the sequenced nucleotide sequence, such as in the form of a quality score (Q-score or simply QS). In such a case, the exclusion of low quality sequence reads is preferably based on the quality scores for the different sequence reads. In more detail, sequence reads having a quality score representing a poor quality in any sequenced nucleotide, such as below a minimum threshold value, are excluded from the continuing processing.

The next step S21 involves sample demultiplex. As mentioned in the foregoing, following the hybridization, ligation and amplification steps, which are conducted in separate containers for each sample or patient, the contents from the amplification steps are mixed and pooled together. However, the present embodiments use patient- or sample-specific barcode sequences. Accordingly, the sequence reads can be demultiplexed, i.e., separated, in step S21 based on the respective barcode sequence in sequenced nucleotide sequences.

In a next optional step S22, the analyzed loci are mapped against reference sequences, i.e., divided based on the genomic context. This loci-mapping thereby divides the sequence reads based on the nucleotide sequences of the target DNA sequences.

A next optional step S23 is performed for allele counting.

Step S24 is performed to estimate the PCR amplification effect per each DNA molecule and to eliminate any duplicate/clone DNA molecules that share the identical 8 nt UMI motif, i.e., all identical copies of cloned amplicons are removed. In an illustrative example, PCR redundancy (amplification effect) was calculated in step S24 as 10, which means that original DNA molecule was first amplified and detected simultaneously 10 times on sequencing flow-cell, for example.

In a next step S25, the final number of original DNA molecules after S24 per locus is counted. The true number of original sequencing reads as determined in step S25 represents the studied allele count without PCR amplification bias. Step S25 completes the data analysis of sequencing reads and provides input to any further statistical analysis, for instance for NIPT studies.

Another aspect of the embodiments relates to a kit for quantifying target DNA sequences 1. The kit comprises *M_{L}* left ligation oligonucleotides 10 comprising, from a 5' end 11 to a 3' end 12, a sequencing read-1 primer site 15, a respective first UMI 14 and a respective sequence 13 complementary to a respective first segment 2 of a target DNA sequence 1. The kit also comprises *M_{R}* right ligation oligonucleotides 20 comprising, from a 5' end 21 to a 3' end 22, a respective sequence 23 complementary to a respective second segment 3 of a target DNA sequence 1, a respective second UMI 24 and a sequencing read-2 primer site 25. *M_{L}* and *M_{R}* are each an integer equal to or larger than two. The respective first UMI 14 is different for each of the *M_{L}* left ligation oligonucleotides 21 and the respective second UMI 24 is different for each of the *M_{R}* right ligation oligonucleotides 20. The kit further comprises a left amplification primer 40 comprising, from a 5' end 41 to a 3' end 42, a first common sequence 43 and the sequencing read-1 primer site 44 and a right amplification primer 50 comprising, from a 5' end 51 to a 3' end 52, a second common sequence 53, a barcode sequence 55 and a ligation oligonucleotide binding site 54 complementary to the sequencing read-2 primer site 25. The kit additionally comprises a sequencing read-1 primer 80 comprising the sequencing read-1 primer site 15 and a sequencing read-2 primer 81 comprising the read-2 primer site 25.

In an embodiment, the kit also comprises a ligating agent, preferably a ligase, and more preferably a DNA ligase, capable of ligating together the 3' end 12 of a left ligation oligonucleotide 10 and the 5' end 21 of a right ligation oligonucleotide 20 while hybridized to a target DNA sequence 1 to form a ligated product 30 comprising two UMIs 31, 32 and hybridized to the target DNA sequence 1.

In an embodiment, the kit also comprises a solid phase 4 having preference for DNA sequences of a length in terms of number of deoxyribonucleotides equal to or larger than a minimum length.

In an embodiment, the 5' end 21 of the *M_{R}* right ligation oligonucleotides 20 comprises a 5' phosphate group.

In an embodiment, the first UMI 14 and the second UMI 24 are each a random nm₂n₃...nₖ sequence, wherein nᵢ, i=1 ...k, is one of A, T, C and G, and k is from 2 up to 6, preferably from 3 up to 5, such as 4.

In an embodiment, the barcode sequence 55 is a P nucleotides sample-specific barcode sequence, wherein P is from 4 up to 16 nucleotides, preferably 4 up to 10 nucleotides, more preferably 6 up to 8 nucleotides, such as 6 nucleotides.

In an embodiment, the first common sequence 43 is one of the P5 sequence (5'-AATGATACGGCGACCACCGA-3', SEQ ID NO: 1) and the P7 sequence (5'-CAAGCAGAAGACGGCATACGAGAT-3', SEQ ID NO: 2) and the second common sequence 53 is the other of the P5 sequence and the P7 sequence.

In an embodiment, the sequencing read-1 primer 80 has the nucleotide sequence of ACACTCTTTCCCTACACGACGCTCTTCCGATCT (SEQ ID NO: 3) and the sequencing read-2 primer 81 has the nucleotide sequence of CTGGAGCTGTCTGCGACTTT (SEQ ID NO: 4). In an embodiment, at least one nucleotide of the sequencing read-2 primer is a locked nucleic acid (LNA) nucleotide.

The quantification method of the embodiments can be used in various applications.

Besides traditional DNA testing, the type of liquid biomarker, denoted as cell-free DNA (cfDNA), of blood plasma is increasing its potential in fetal Non-Invasive Prenatal Testing (NIPT) and cancer diagnostics. cfDNA circulates in blood and originates from apoptotic cells or cells from placenta in case of pregnancy. In NIPT and cancer diagnostics, specific cfNDA is detectable already in early phase of tumor recurrence or pregnancy, providing robust, sensitive and flexible blood genetic test alternatives in comparison to insensitive tumor imaging or risky and unpleasant invasive prenatal testing.

Still, the testing of new type of biomarkers, like cfDNA, meet challenges for method sensitivity, robustness and price, which have so far limited its practical clinical usability. The present embodiments teach a commercially viable method and kit to fill the current methodological gap between available nucleic acid detection platforms.

The method and kit can also be used as an extremely sensitive screening tool to detect early tumor recurrence or relapse. The cancer-specific cfDNAs are analyzed to detect previously characterized and personalized tumor-specific mutations from the patient's blood, which is indicative for cancer relapse and the need for treatment already in the early phase of recurred cancer.

The method and kit are quantitative, providing the absolute number of analyzed biomolecules from the mixed pool, and eliminating PCR-induced bias even in case of limited starting material. The method and kit are highly competitive in throughput, price and potential of automatization, when compared to existing platforms and technologies.

The method and kit can be used for analyzing target loci over targeted chromosomes for aneuploidy detection. The method and kit enable Targeted Allele Counting by sequencing (TAC-seq™) through specific probe hybridization and thermostable ligation. The formed amplified product contains the UMI sequences to minimize PCR-induced bias and enables absolute biomarker molecule and allele counting.

The UMIs of the ligation oligonucleotides make each amplified product in a population distinct and enable absolute counting of original target DNA sequences. The UMI principle enables amplification while not requiring detecting each original target DNA sequence or keeping track of the number of copies made. UMI is a random sequence of certain number of nucleotides that are involved to the primary step of SOI analysis. The effective usage of UMI motif in ligation assays is not described so far. UMI provides a dimension of more sensitivity to ligation-based assays compared to existing solutions, enabling absolute molecule counting and more precise computing algorithms for NIPT, gene expression or DNA mutation detection.

Possible applications include profiling endometrium receptivity, preeclampsia and asthma patients for critical gene expression signatures. A further application is NIPT detection of fetal aneuploidies. cfDNA extracted from blood plasma of pregnant women is analyzed. The method and kit may also be used in tumor specific mutation screening to detect possible tumor relapse. The tumor biopsy of each patient is genetically profiled by cancer-panel sequencing or alternatively whole-exome sequencing after surgery and the cancer-specific mutations are selected for testing.

### EXAMPLES

As accurate and cost-effective detection of nucleic acid biomarkers is still challenging in biomedical diagnostics. A targeted allele counting by sequencing (TAC-seq™) assay has been developed to quantify, among others, cell-free DNA. High stringency is guaranteed by specific ligation-dependent probes combined with unique molecular identifier. Feasibility of the assay for targeted transcriptomic and cell-free DNA-based chromosome aneuploidy analysis is demonstrated.

Biomarker detection is a basic technique in biomedicine, allowing dynamic observation of physiological conditions. Both PCR and sequencing are mainstream methods to analyze circulating cell-free DNA (cfDNA) as a biomarker. Although protocols for cfDNA biomarkers detection are somewhat different, the intersection is the target nucleic acid itself. Either way the studied material needs manipulations prior sequencing, introducing technical bias to the analysis. Therefore, novel methods that maintain sensitivity and throughput, simultaneously lowering preparation bias together with running costs, are desired for routine biomarker detection.

### TAC-seq™ assay principle

A method for targeted molecule counting analysis of cfDNA is described, see Figs. 1A-1E. The studied alleles are defined by a single 54 bp specific region 2, 3 that is equally covered by two TAC-seq™ probes 10, 20. Both ligation oligonucleotides 10, 20 have a specific sequence complementary 13, 23 to target DNA 1, and a unique molecular identifier (UMI) 14, 24 that is divided between both ligation oligonucleotides 10, 20. UMIs 14, 24 are beneficial only if the number of UMI combinations (8 bp UMI provides 4⁸ = 65,536 variants, for example) is substantially larger than the sum of target DNA molecules 1 in the studied sample. Based on calculation, one genomic allele is present in approximately 3,000 copies in 10 ng cfDNA, being notably lower than 8 bp UMI combinations and allowing extremely precise counting of cfDNA molecules as well as SNP/mutation detection. However, expression of transcriptional biomarkers is highly variable necessitating high number of UMI combinations and scrutinized data analysis to avoid UMI-caused saturation.

Key unique features of the TAC-seq™ assay, compared to existing ligation-based assays, are (i) the use of UMI motif 14, 24 in the ligation oligonucleotides 10, 20 to enable targeted DNA molecule 1 counting, and (ii) a dilution-free protocol. In essence, the TAC-seq™ assay is a hybridization and ligation-based method. Once specific hybridization is completed, a thermostable ligase is introduced, catalyzing the formation of a phosphodiester bond between the 5'-phosphate and the 3'-hydroxyl of two ligation oligonucleotides 10, 20. In order for the ligation oligonucleotides 10, 20 to be ligated both ligation oligonucleotides 10, 20 need to be hybridized with high specificity. Ligated ligation oligonucleotides 10, 20 in complex with template DNA 1 are captured by paramagnetic carboxylated beads 4 allowing buffer exchange and disposal of the majority of unbound ligation oligonucleotides 10, 20, especially right ligation oligonucleotides 20 that could generate abundant 80 bp by-product during amplification, see Figs. 1D and 1E. The following PCR introduces individual barcodes 55 and universal sequences 43, 54 for sequencing. The TAC-seq™ assay is a single-tube assay that retains all biomarker molecules since the entire pipeline from material extraction to sequencing, allowing manual or automated analysis with minimum losses.

### Sampling

The study was approved by Research Ethics Committee of the University of Tartu (246/T-21 and 221/M-31). Endometrial biopsies for RNA assays were collected from healthy volunteers with proven fertility as previously described [13]. Genomic DNAs from GM01359 and GM04616 cell lines (NIGMS Human Genetic Cell Repository at the Coriell Institute of Medical Research (USA)) were extracted using DNeasy Blood and Tissue kit (Qiagen). To mimic cfDNA, the genomic DNAs were fragmented to 150-200 bp using Covaris M220 Focused-ultrasonicator (Thermo Fisher) with following settings: 50 µl volume, 10% duty cycle, 75 W peak incident power, 200 cycles per burst and 360 s. Concentrations were quantified by Qubit dsDNA HS (Thermo Fisher).

### Biomarker selection and probe design

The biomarkers of endometrial receptivity were selected from the publication [13]. Briefly, nine studies incorporating 164 endometrial samples from fertile women were included to the meta-analysis using Robust Rank Aggregation method. Altogether, 57 mRNAs were identified as potential endometrial receptivity biomarkers and used in this study for to distinguish the pre-receptive and receptive endometrial samples. A pair of TAC-seq™ probes (left and right ligation oligonucleotides) was designed for every targeted gene using probe design software (https://hindrek.shinyapps.io/probe_design/). Both the left and right ligation oligonucleotides consisted of a specific sequence (27 bp), a UMI (4 bp) and a left probe universal sequence ACACGACGCTCTTCCGATCT (SEQ ID NO: 5) or a right probe universal sequence CTGGAGCTGTCTGCGACTTT (SEQ ID NO: 4). Each pair targeted the coding sequence in the Consensus Coding Sequence Set (CCDS). For genes without CCDS, the most likely transcript was chosen manually from the Ensembl 87 database. The selection of the target sequence was based on criteria - the unique sequences were ranked by the distance from the 3'-end of the transcript. Routine genetic testing probes were preferentially designed close to transcript's 3'-end to minimize the effect of possible RNA degradation caused by sampling and handling. Also, probe-specific regions were filtered by GC-content for optimal melting temperature. The overall GC-content of a ligation oligonucleotide had to be between 40-60% and the GC-content of the adjacent ends (4 bp) was up to 50%. In addition, ligation oligonucleotides with inter- or intra-complementarity issues were excluded from the selection. The software has an option to design the ligation oligonucleotides close to transcript's 5' end, depending on the assay. ERCC spike-in 22 probes were design based on previous description.

Chromosome 2 and chromosome 21 loci were selected from whole genome k-mer database (http://bioinfo.ut.ee/FastGT) where k-mers overlapping with known polymorphisms (dbSNP build id 149) were first removed and remaining candidates were used as an input for BLAST 2.4.0+ (task blastn) with database version of GRCh38 (GCA_000001405.15). All reads with more than one exact match were also removed, following the concatenation of overlapping regions. The regions were converted to sequences with UCSC Genome Browser Gateway. Altogether 118 specific probe pairs over studied chromosome 2 and 21 were selected after above described design, ensuring equal coverage over entire chromosome.

### Library preparation for ERCC quantification

Non-skirted low profile Plate (Thermo Fisher) was used with domed cap strips (Thermo Fisher). ERCC Spike-In Mix 1 (Life Technologies) was first 10x and next additional 100x diluted with water. Aliquots, each containing 1.3 µl of 1,000x dilution, were stored at-70°C until use. Next, 199 µl of water was added to 1.3 µl aliquot and pipetted through. One microlitre of diluted ERCC spike-in content, as a template for each individual library was added to 2 µl denaturation buffer, containing 5 mM Tris-HCI (pH 7.0) (Sigma-Aldrich), 1 mM dNTP mixture (Thermo Fisher), 400 nM Oligo-T30 primer and 0.05% Triton X-100 (Sigma-Aldrich). Reaction was mixed by pipetting and centrifuged briefly. RNA was denatured by 1 min at 80°C and placed on ice. After that, reverse transcriptase (RT) master mix containing 100 mM Tris-HCI (pH 8.5) (Sigma-Aldrich), 2.5 M betaine (Sigma-Aldrich), 150 mM KCI (Sigma-Aldrich), 10 mM DTT (Sigma-Aldrich), 15 mM MgCI (Sigma-Aldrich), 4 U RiboLock RNase inhibitor (Thermo Fisher) and 20 U Maxima H Minus Reverse Transcriptase (Thermo Fisher) was prepared. The content was mixed on vortex and centrifuged briefly. Two microlitres of RT master mix was added to previously denatured RNA (3 µl). All RT pipetting steps were performed on ice. Complementary DNA synthesis was performed by 30 min at 42°C, following 5 min at 85°C for RT inactivation.

Twenty two TAC-seq™ probes (ligation oligonucleotides) targeting ERCC spike-in molecules were previously mixed together from 100 µM stock solutions and creating a 100 µM oligo pool. The mixture was diluted to 5 µM by water and stored at -20°C. Once cDNA synthesis was completed, one microlitre of 5 µM TAC-seq™ probe mixture was added to RT mixture. The content was mixed on vortex and centrifuged briefly. Strip tubes were placed on thermocycler, denatured 1 min at 98°C and following 1 h at 60°C to enable specific cDNA and TAC-seq™ probe hybridization. After hybridization, thermostable ligase reaction mixture was added on thermocycler, keeping a constant (60°C) hybridization temperature. The cycler lid was opened and strip caps were removed. Five microlitres of Taq DNA ligase master mix, containing 2x Taq DNA ligation buffer (New England Biolabs, NEB) and 1 U Taq DNA ligase (NEB, cat no M0208) were added to each individual reaction tube and mixed by pipetting. The strip tubes were not removed from 60°C thermocycler to avoid self- and mispairing of TAC-seq™ probes. Ligation reaction was stopped after 20 min incubation by placing reaction tubes on ice.

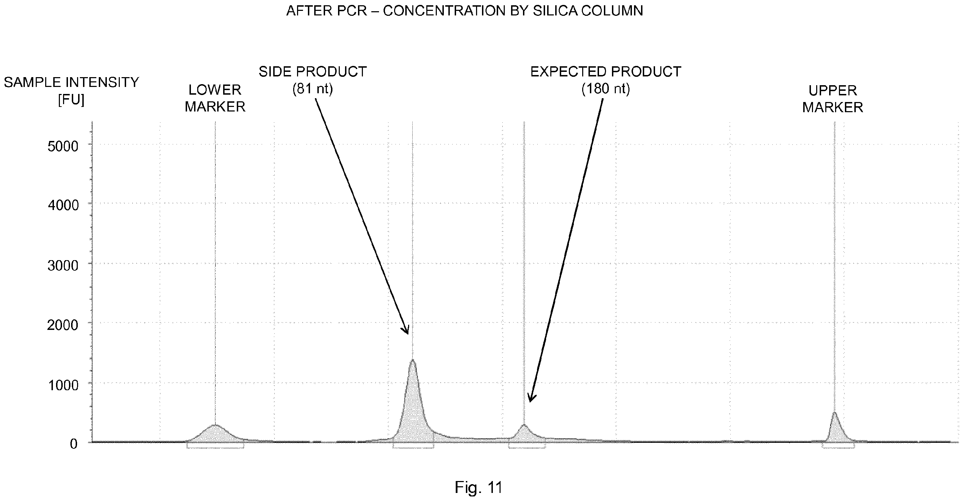

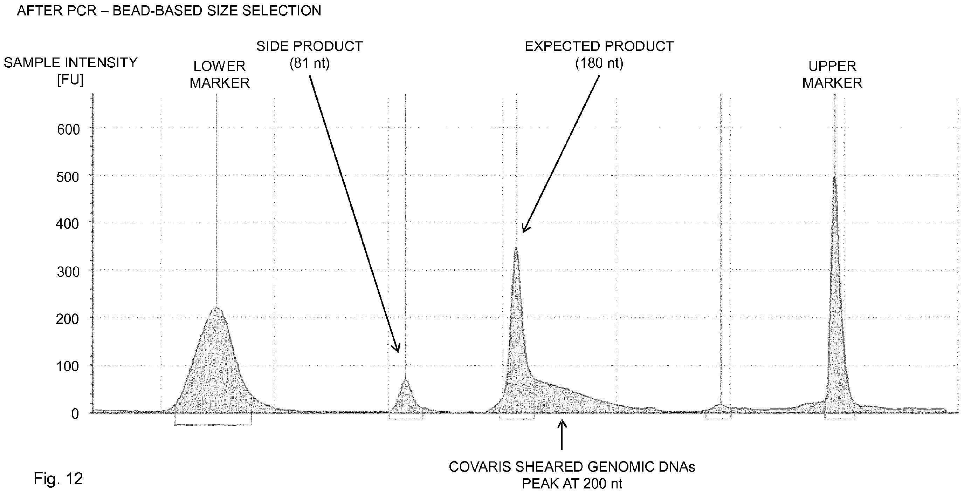

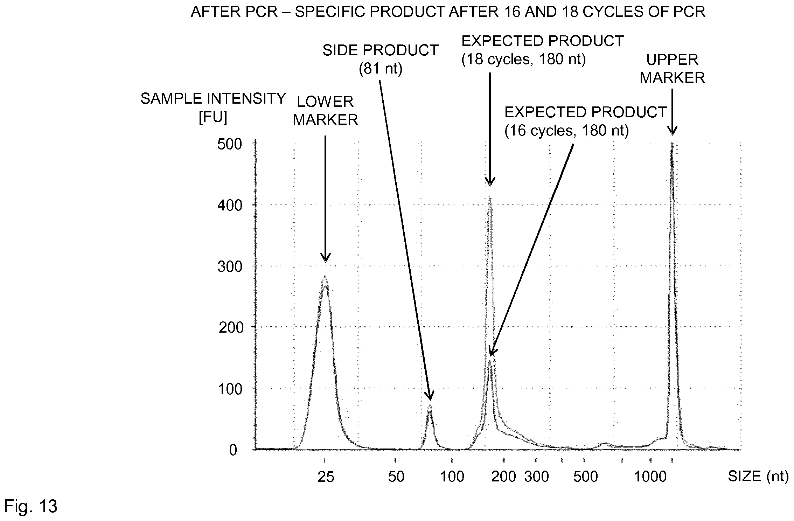

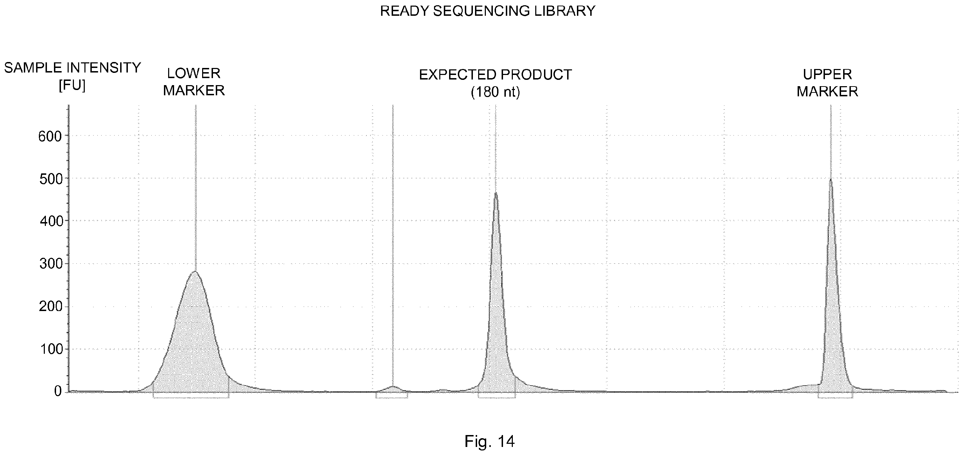

Fifteen microlitres of previously combined Dynabeads MyOne Carboxylic Acid beads (2 µl) (Thermo Fisher) and 13 µl of capture buffer (30% PEG-6000 (Sigma-Aldrich), 2 M NaCl (Sigma-Aldrich), 5 mM Tris-HCI (pH 7.5) (Sigma-Aldrich), 10 mM EDTA (Sigma-Aldrich) and 0.02% Tween-20 (Sigma-Aldrich)) was added to cooled ligated sample. The content was mixed by pipetting. The capture was carried out for ten minutes at RT, and after that the samples were placed on DynaMag-96 Side Magnet (Thermo Fisher) holding 8-well strip tubes on VersiPlate Frame (Thermo Fisher). The clean supernatant was removed. The beads on magnet were washed once with 50 µl fresh 80% ethanol. Ethanol was removed by pipetting, and the clean pellet, without ethanol drops, was dried for two minutes. Once beads were dry, strip tubes were removed from the magnet and 18 µl of PCR master mix was added directly to beads. The master mix contained 1× proofreading HOT FIREPol Blend Master Mix (SolisBiodyne, Tartu, Estonia) and 500 nM TAC-seq™ Left (left amplification primer). In addition to universal TAC-seq™ Left, 16 different TAC-seq™ barcode oligonucleotides (right amplification primers) were used to introduce a six-nucleotide barcode to each studied sample (Table 1). Two microlitres of 5 µM TAC-seq™ Barcoded 1-16 primers were added individually to each PCR reaction. Strip tubes were closed with clean domed caps, mixed on vortex until all beads were suspended. The reaction was incubated at 95°C for 12 min, followed by two cycles of 95°C for 20 s, 57°C for 60 s and 72°C for 20 s. In addition, 16 cycles of 95°C for 20 s, 65°C for 20 s and 72°C for 20 s with a final extension at 72°C for 1 min using the default ramp speed of the T100 cycler (Bio-Rad) were performed. PCR products were pooled together into 1.5 ml tube. The pooled tube was placed on magnet to remove carboxylated beads before the following column purification. Clear supernatant was purified with DNA Clean & Concentrator-5 column (Zymo Research) and eluted with 50 µl EB. The library was size-selected using AMPure XP beads (Beckman Coulter) in a single-step selection to remove 81 bp linear PCR double-stranded by-product. 50 µl beads were added to 50 µl of the purified PCR product, incubated for 5 min at room temperature and captured by a magnet for 3 min. After incubation on magnet, the supernatant was discarded and the remaining beads were centrifuged at 500xg for 10 seconds. After centrifugation, the beads were placed again on the magnet and all remaining supernatant was removed. The beads were eluted directly without ethanol washing in 25 µl of EB and incubated for 1 min at RT. Finally, the eluted library was transferred to a clean tube after 1 min incubation on the magnet. The library (single 180 bp product, Figs. 11-14) was visualized on a TapeStation High Sensitivity D1000 ScreenTape (Agilent Technologies) and quantified using KAPA Library Quantification Kit (Roche).

### Library preparation to detect RNA biomarkers

RNA biomarker libraries were prepared as described above with the following modifications. One microlitre of total-RNA sample was used. The RIN values 7.7 - 9.6 were quantified by Qubit (Invitrogen) and diluted to concentration 90 ng/µl. RT master mix contained 1 µl of 1:50,000 of ERCC RNA Spike-In Mix 1 (Life Technologies) dilution for technical normalization. 64-plex TAC-seq™ probe set, containing 57 biomarker genes [13] and seven ERCC spike-ins (ERCC-00085; 00170; 00019; 00131; 00092; 00108 and 00004) were used to generate a library. Five micromolar probe mixtures from 100 µM oligo stock were created as described previously. PCR was performed in total using 12 cycles, following 2+10 principle (details above).

### Library preparation to detect trisomy from cell-free DNA

Ten nanograms of acoustically sheared (Covaris) cell-free like DNAs were combined to create excess rates of chromosome 21 above diploidy level, from 5 till 30% extra that mimic 5 to 30% fetal fraction. 100% fraction is the GM04616 cell line DNA with trisomy 21. Each concentration was performed as duplicate. Samples were pipetted into strip tubes, adding one microlitre of 5 µM TAC-seq™ probe mixture and 1 µl 10x hybridization buffer, containing 100 mM Tris-HCI (pH 7.5) (Sigma-Aldrich), 500 mM KCI (Sigma-Aldrich), 0.2% Tween-20 (Sigma-Aldrich) and 0.1 mM EDTA (Sigma-Aldrich). The final hybridization volume was 12 µl. The content was mixed on vortex and centrifuged briefly. Strip tubes were placed on thermocycler, denatured 2 min at 98°C and following 1 h at 60°C for hybridization. After hybridization, thermostable ligase reaction master mix was added on thermocycler, keeping constant (60°C) hybridization temperature. Following, 2.5 µl Taq DNA ligase mixture, containing 1.5 µl 10× Taq DNA ligation buffer (NEB) and 1 U Taq DNA ligase (NEB) was added to each individual reaction tube and mixed by pipetting. Ligation reaction was stopped after 20 min incubation by placing reaction tubes on ice.

Twenty five microlitres of previously combined Dynabeads MyOne Carboxylic Acid beads (3 µl) (Thermo Fisher) and 22 µl of capture buffer as described above. Ligated TAC-seq™ probes were amplified as previously described using 2+19 PCR cycles.

### Reference RNA-sequencing and data analysis

The total-RNA samples with concentration at least 200 ng/ml and RIN >8 were used for endometrium receptivity cDNA library construction. Libraries were generated from ∼1 pg of total-RNA using TruSeq Stranded Total RNA (Illumina) protocol. Libraries were normalized, pooled and sequenced by Illumina HiSeq2500 instrument producing 100 cycles paired-end reads. The RNA-seq data was analyzed as previously described [13]. Heatmaps of the results were generated using the pheatmap package implemented in R. For plotting, counts per million (CPM) values provided by edgeR were log-transformed, using the transformation log(CPM+1) to facilitate graphical presentation of the results.

### TAC-seq™ sequencing

The ERCC spike-in library was sequenced by Illumina NextSeq500 high output 75 cycles kit and 2 pM library concentration. The library was sequenced using 88 nt single-read protocol that was primed by Illumina Read1 (HP10) primer. The entire construct was 88 nt. The second, RNA biomarker based endometrium receptivity set was sequenced using read-2 sequencing primer (CTGGAGCTGTCTGCGACTTT, SEQ ID NO: 4, underlined are LNA nucleotides) by MiSeq Reagent Kit v3 (150 cycles) using 14 pM library loading concentration. Read-2 primer avoids low-diversity region and significantly improved the chastity filter (pass-filter) outcome. In total 62 nt Read-1 and 6 nt read-2 (barcode nucleotides) were sequenced. Cell-free DNA library, was analyzed by NextSeq500 instrument, using read-1 and read-2 primers, 1.8 pM loading concentration, 63 nt for read-1 and six nt for read-2 configuration.

### TAC-seq™ data analysis

ERCC spike-in reads were trimmed to construct length of 88 nt and demultiplexed by barcodes (6 nt) allowing one mismatch. For this experiment only read-1 primer was used to analyze both UMIs, SOI and barcode sequence with long 88 nt read. Demultiplexed reads were further trimmed to length of 62 nt and 4 nt of UMI at the end of the read was inserted after 4 nt of the UMI at the start of the read. Reads with UMI that contained unallocated nucleotides, were disregarded. Resulting reads per sample were demultiplexed again using target regions of the genes (54 nt) allowing up to 5 mismatches. Total read counts and unique molecule counts were calculated at different threshold of UMI.

Gene expression reads were processed as above. To reduce potential sequencing error accumulating at UMI motif, only reads appearing at least twice were counted as unique molecules. Each sample was normalized to counts per million (CPM) using edgeR (version 3.18.1) package in R (version 3.4.1) and log-transformed using the log10 (CPM+1) transformation to reduce skewness. In addition to biomarker genes spike-in molecules were included. The normalization procedure was based on the published formula21 that was further adjusted for read count data.

Genomic DNA data quality control and the reads preprocessing were performed as previously described herein. Loci that were 1.5 interquartile ranges (IQRs) below the first quartile or above the third quartile were named as outliers and removed. As we constantly detected slightly higher molecule counts in chromosome 2 compared to chromosome 21 in euploid samples, chromosome-specificity molecule counts was applied. Mean molecule counts of chromosome 2 and chromosome 21 (∼1.08) using the euploid samples were calculated and used for normalization.

### ERCC spike-in

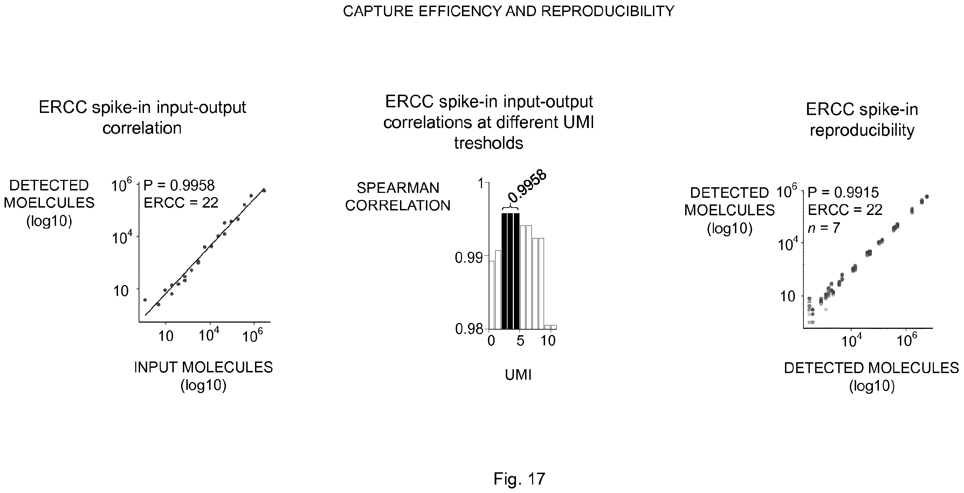

External RNA Controls Consortium (ERCC) spike-in controls were used to observe the technical performance. Twenty two synthetic spike-ins were selected and detected under different criteria, indicating highest Spearman's correlation (p=0.9958) between targeted and detected molecules at UMI threshold 2-4 (Fig. 17 left and middle) because potential polymerase caused bias at 8 bp UMI site should be taken into account. The UMI threshold is justified and applicable only at high coverage sequencing, where the raw read number is significantly higher than the UMI corrected outcome. Over seven technical replicates, the average 1.518 M ± 6.74% s.d. raw read count per replica dropped to 57 K ± 5.64% s.d. after UMI correction, having 102x redundancy at UMI threshold 3. Simultaneously, the replicates demonstrate excellent reproducibility (p=0.9915, Spearman's correlation, Fig. 17 right) over seven studied samples. Although TAC-seq™ sensitivity is not related with natural RNA capping and template switching during cDNA synthesis, ERCC spike-in's ability to form secondary structures is potentially different compared to transcripts. However, as spike-in concentrations are known, it is still the best tool to validate different protocols and track the quality performance, enabling normalization over multiple samples.

### TAC-seq™ expression assay

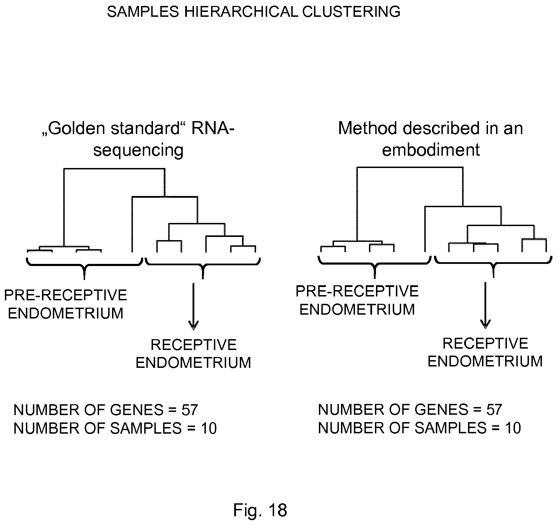

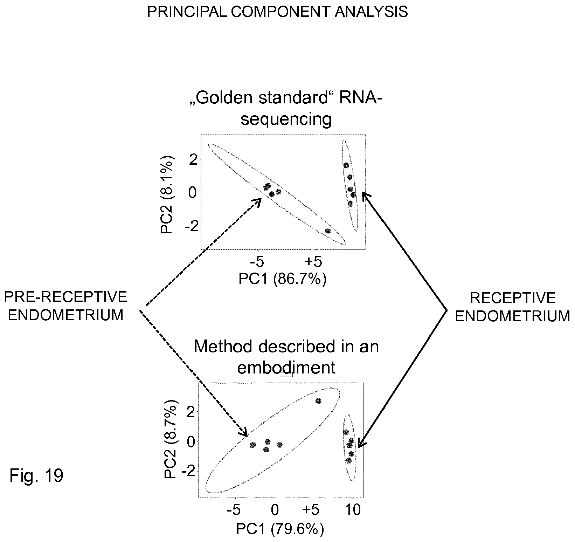

Genetic testing prototype to analyze 57 human endometrial receptivity biomarkers that were previously chosen [13] was created. The endometrial biopsies were analyzed by the TAC-seq™ assay and compared with 'golden standard' RNA-sequencing (Fig. 18). The TAC-seq™ assay showed 100% performance across selected 57 genes and the same trend was confirmed by previous 22-plex ERCC spike-in experiment where all TAC-seq™ probes worked faultlessly. The deep sequencing revealed the limit of eight nucleotide UMI, detecting six genes close to saturation level. The border could be easily improved by adding extra UMI nucleotides to probe oligonucleotides or simply producing less reads. Expression profiles from the TAC-seq™ and RNA-seq assay clustered highly similarly between studied samples (Fig. 18). The TAC-seq™ sequencing ensured 25.71 M ± 23.87 s.d. reads per sample after UMI correction, having 10x redundancy. The studied samples were clustered based on heatmap and PCA plot comparison (Fig. 19), confirming that the TAC-seq™ assay has as sensitive transcriptome biomarker detection method as full transcriptome RNA-sequencing.

### Cell-free based trisomy detection

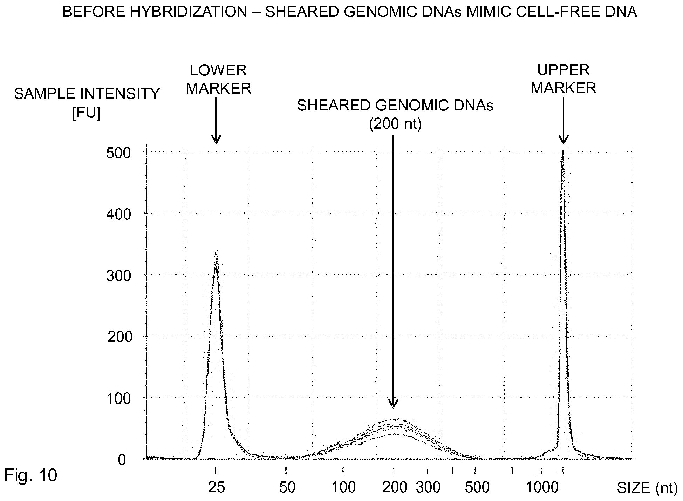

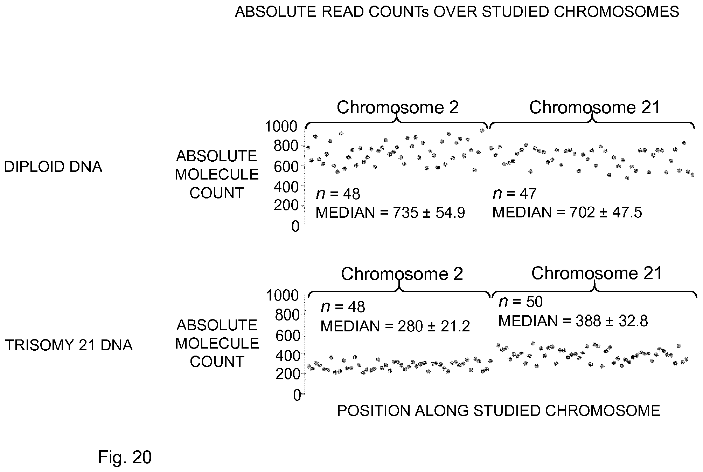

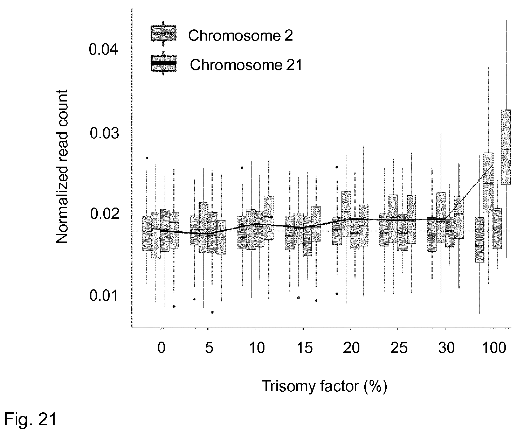

Genomic DNA (gDNA) was tested by using chromosome 21 aneuploid cell line in combination with diploid gDNA where chromosome 2 was used as a reference. The gDNAs were acoustically sheared (Fig. 10) to mimic the nature of cfDNA, mixed together in a way to create 5-30% trisomy factor and analyzed by altogether over 118 loci of both chromosomes. Unique molecule counts along chromosome 2 and 21 after interquartile range filtering present unequal median values of chromosome 2 (735 ± 54.9) and chromosome 21 (702 ± 47.5) (Fig. 20) in diploid DNA. However, aneuploidy DNA revealed clear increase of chromosome 21 read count, being one third higher than reference chromosome (Fig. 20). As reference molecule counts had on average 1.08× higher values than chromosome 21 in two diploid reference samples, normalized molecule counts were calculated for each sample and plotted as chromosome-wise in different trisomic cell proportions (Fig. 21). Different trisomy factor proportions were used to imitate the range of "fetal fraction" in case of trisomy 21 fetus. The normalized molecule counts of chromosome 21 showed positive correlation as the trisomic cell proportion increases since 10% of target excess.

### Final conclusion

Here we demonstrate targeted allele counting by sequencing method that compiles first time specific ligation-based assay with UMIs into automatization compatible single-tube protocol. The method uses off-the-shelf reagents, providing low-cost detection method for cDNA biomarkers as well as cfDNA. We foresee application of the method in non-invasive prenatal trisomy screening (NIPT), in tumor early relapse screening and in robust RNA biomarker detection.

**Table 1 - probes**

| Probe | SEQ ID NO: | Sequence (5'-3') |
|---|---|---|
| Oligo- T30 | 6 | TTTTTTTTTTTTTTTTTTTTTTTTTTTTTT |
| TAC-seq™ Left | 7 | |
| Barcode-seq primer LNA | 4 | CTGGAGCTGTCTGCGACTTT |
| TAC-seq™ Barcode 1 | 8 | CAAGCAGAAGACGGCATACGAGATGATCTGAAAGTCGCAGACAGCTCCAG |
| TAC-seq™ Barcode 2 | 9 | CAAGCAGAAGACGGCATACGAGATGCCTAAAAAGTCGCAGACAGCTCCAG |
| TAC-seq™ Barcode 3 | 10 | CAAGCAGAAGACGGCATACGAGATCGTGATAAAGTCGCAGACAGCTCCAG |
| TAC-seq™ Barcode 4 | 11 | CAAGCAGAAGACGGCATACGAGATTGGTCAAAAGTCGCAGACAGCTCCAG |
| TAC-seq™ Barcode 5 | 12 | CAAGCAGAAGACGGCATACGAGATATTGGCAAAGTCGCAGACAGCTCCAG |
| TAC-seq™ Barcode 6 | 13 | CAAGCAGAAGACGGCATACGAGATCTGATCAAAGTCGCAGACAGCTCCAG |
| TAC-seq™ Barcode 7 | 14 | CAAGCAGAAGACGGCATACGAGATGTAGCCAAAGTCGCAGACAGCTCCAG |
| TAC-seq™ Barcode 8 | 15 | CAAGCAGAAGACGGCATACGAGATTACAAGAAAGTCGCAGACAGCTCCAG |
| TAC-seq™ Barcode 9 | 16 | CAAGCAGAAGACGGCATACGAGATATCAGTAAAGTCGCAGACAGCTCCAG |
| TAC-seq™ Barcode 10 | 17 | CAAGCAGAAGACGGCATACGAGATAGGAATAAAGTCGCAGACAGCTCCAG |
| TAC-seq™ Barcode 11 | 18 | CAAGCAGAAGACGGCATACGAGATTAGTTGAAAGTCGCAGACAGCTCCAG |
| TAC-seq™ Barcode 12 | 19 | CAAGCAGAAGACGGCATACGAGATATCGTGAAAGTCGCAGACAGCTCCAG |
| TAC-seq™ Barcode 13 | 20 | CAAGCAGAAGACGGCATACGAGATTGAGTGAAAGTCGCAGACAGCTCCAG |
| TAC-seq™ Barcode 14 | 21 | CAAGCAGAAGACGGCATACGAGATGCCATGAAAGTCGCAGACAGCTCCAG |
| TAC-seq™ Barcode 15 | 22 | CAAGCAGAAGACGGCATACGAGATTGTTGGAAAGTCGCAGACAGCTCCAG |
| TAC-seq™ Barcode 16 | 23 | CAAGCAGAAGACGGCATACGAGATAGCATCAAAGTCGCAGACAGCTCCAG |
| Ligation probe-L | 24 | ACACGACGCTCTTCCGATCTNNNNnnnnnnnnnnnnnnnnnnnnnnnnnnn |
| Ligation probe-R | 25 | nnnnnnnnnnnnnnnnnnnnnnnnnnnNNNNCTGGAGCTGTCTGCGACTTT |

| | | |
|---|---|---|
| NNNN are four random nucleotides used as UMI, LNA bases are underlined. The 5'-end of the right ligation oligonucleotides was phosphorylated. | | |

The embodiments described above are to be understood as a few illustrative examples of the present invention. It The scope of the present invention is, however, defined by the appended claims.

### REFERENCES

[1] WO 96/15271
[2] WO 97/45559
[3] Nucleic Acids Res. 2002, 30(12): e57
[4] J Pathol. 2006, 209(2): 220-230
[5] Prenat Diagn. 2012, 32(1): 3-9
[6] Technology Spotlight: ILLUMINA® Sequencing, Illumina Sequencing Technology, Highest data accuracy, simple workflow, and a broad range of applications. (https://studylib.net/doc/8828848/illumina-sequencing-technology retrieved on December 14, 2016)
[9] PLoS One. 2017, 12(5):e0178302
[10] US 2016/0068886
[11] WO 2016/123154
[12] WO 2013/106807
[13] Sci Rep 2017, 7(1): 10077
[14] Eur J Hum Genet 2015, 23(10): 1286-1293

## Claims

1. A method for quantifying target deoxyribonucleic acid (DNA) sequences (1), said method comprising the steps of:
contacting (S1), in each container of *N* separate containers, *N* being an integer equal to or larger than two, target DNA sequences (1), under hybridization conditions, with *M_{L}* left ligation oligonucleotides (10) comprising, from a 5' end (11) to a 3' end (12), a sequencing read-1 primer site (15), a respective first unique molecular identifier (UMI) (14) and a respective sequence (13) complementary to a respective first segment (2) of a target DNA sequence (1) and *M_{R}* right ligation oligonucleotides (20) comprising, from a 5' end (21) to a 3' end (22), a respective sequence (23) complementary to a respective second segment (3) of a target DNA sequence (1), a respective second UMI (24) and a sequencing read-2 primer site (25), wherein *M_{L}* and *M_{R}* are each an integer equal to or larger than two, said respective first UMI (14) being different for each of said *M_{L}* left ligation oligonucleotides (21) and said respective second UMI (24) being different for each of said *M_{R}* right ligation oligonucleotides (20);
adding (S2), in each container of said N separate containers, a ligating agent capable of ligating together said 3' end (12) of a left ligation oligonucleotide (10) and said 5' end (21) of a right ligation oligonucleotide (20) while hybridized to a target DNA sequence (1) to form a ligated product (30) comprising two UMIs (31, 32) and hybridized to said target DNA sequence (1);
immobilizing (S3), in each container of said N separate containers, said ligated product (30) in complex with said target DNA sequence (1) onto a solid phase (4) having preference for DNA sequences of a length in terms of number of deoxyribonucleotides equal to or larger than a minimum length and removing a supernatant;
amplifying (S4), in each container of said *N* separate containers, said ligated product (30) in presence of a left amplification primer (40) comprising, from a 5' end (41) to a 3' end (42), a first common sequence (43) and said sequencing read-1 primer site (44) and a right amplification primer (50) comprising, from a 5' end (51) to a 3' end (52), a second common sequence (53), a barcode sequence (55) and a ligation oligonucleotide binding site (54) complementary to said sequencing read-2 primer site (25) to form an amplified product (60) comprising two UMIs (65A, 65B) and one barcode sequence (68);
mixing (S5) together the content of said *N* separate containers;
sequencing (S6) at least a respective portion of said amplified products (60) by addition of a sequencing read-1 primer (80) comprising said sequencing read-1 primer site (15) and a sequencing read-2 primer (81) comprising said read-2 primer site (25) to form respective sequence reads comprising at least nucleotide sequences of two UMIs (65A, 65B), one barcode sequence (68) and a target DNA sequence (66);
demultiplexing (S7) said sequence reads based on nucleotide sequences of said barcode sequences (68);
mapping (S8) said demultiplexed sequence reads to known DNA sequences based on nucleotide sequences of said target DNA sequence (66); and
quantifying (S9) unique target DNA sequences (1) in said *N* containers based on said demultiplexed and mapped sequence reads and based on nucleotide sequences of said UMIs.

2. The method according to claim 1, wherein adding (S2) said ligating agent comprises adding (S2), in each container of said *N* containers, a ligase to ligate together said 3' end (12) of said left ligation oligonucleotide (10) and said 5' end (21) of said right ligation oligonucleotide (20) while hybridized to said target DNA sequence (1) to form said ligated product (30).

3. The method according to claim 1 or 2, wherein immobilizing (S3) said ligated product (30) comprises immobilizing (S3), in each container of said *N* containers, said ligated product (30) hybridized to said target DNA sequence (1) onto magnetic beads (4) having preference for DNA sequences of a length in terms of number of base pairs equal to or larger than 100 base pairs.

4. The method according to any of the claims 1 to 3, wherein amplifying (S4) said ligated product (30) comprises performing (S4), in each container of said N containers, 2 to 30 cycles, preferably 10 to 20 cycles of amplification of said ligated product (30) in presence of said left amplification primer (40) and said right amplification primer (50) to form said amplified product (60).

5. The method according to any of the claims 1 to 4, further comprising performing (S10) size-based separation of said mixed amplified products (60) from any side-product (70) produced in said amplification.

6. The method according to claim 5, wherein performing (S10) said size-based separation comprises:
contacting (S11) said mixed amplified product (60) with magnetic beads having preference for double-stranded DNA sequences of a target length interval, wherein a length of said amplified product (60) falls within said target length interval;
separating (S12) said magnetic beads from non-bound nucleotide sequences (70); and
retrieving (S13) said amplified product (60) from said magnetic beads.

7. The method according to any of the claims 1 to 6, wherein sequencing (S6) said at least a portion of said amplified product (60) comprises *in situ* sequencing (S6) said at least a portion of said amplified product (60) immobilized onto a solid support based on said first common sequence (63) and/or said second common sequence (69) using read-1 primer (80) and said read-2 primer (81).

8. The method according to any of the claims 1 to 7, wherein demultiplexing (S7) said sequence reads comprises dividing said sequence reads into groups having a same nucleotide sequence of said barcode sequences (68) with at most n mismatches allowed for nucleotide sequences of barcode sequences (68) in a same group, n is zero or one.

9. The method according to any of the claims 1 to 8, wherein mapping (S8) said demultiplexed sequence reads comprises dividing demultiplexed sequence reads into groups having a same nucleotide sequence of said target DNA sequence with at most m mismatches allowed for nucleotide sequences of target DNA sequences in a same group, m is an integer equal to or larger than 0 but no larger than ten, preferably no larger than five.

10. The method according to any of the claims 1 to 9, wherein quantifying (S9) unique target DNA sequences comprises quantifying (S9) unique target DNA sequences based on determining a number of target DNA sequences in said *N* containers having a nucleotide sequence with at most m mistmatches, having a nucleotide sequence of said barcode sequence with at most n mismatches and having different combinations of said first UMI and said second UMI.

11. A kit for quantifying target deoxyribonucleic acid (DNA) sequences (1), said kit comprises:
*M_{L}* left ligation oligonucleotides (10) comprising, from a 5' end (11) to a 3' end (12), a sequencing read-1 primer site (15), a respective first unique molecular identifier (UMI) (14) and a respective sequence (13) complementary to a respective first segment (2) of a target DNA sequence (1);
*M_{R}* right ligation oligonucleotides (20) comprising, from a 5' end (21) to a 3' end (22), a respective sequence (23) complementary to a respective second segment (3) of a target DNA sequence (1), a respective second UMI (24) and a sequencing read-2 primer site (25), wherein *M_{L}* and *M_{R}* are each an integer equal to or larger than two, said respective first UMI (14) being different for each of said *M_{L}* left ligation oligonucleotides (21) and said respective second UMI (24) being different for each of said *M_{R}* right ligation oligonucleotides (20);
a left amplification primer (40) comprising, from a 5' end (41) to a 3' end (42), a first common sequence (43) and said sequencing read-1 primer site (44);
a right amplification primer (50) comprising, from a 5' end (51) to a 3' end (52), a second common sequence (53), a barcode sequence (55) and a ligation oligonucleotide binding site (54) complementary to said sequencing read-2 primer site (25);
a sequencing read-1 primer (80) comprising said sequencing read-1 primer site (15); and
a sequencing read-2 primer (81) comprising said read-2 primer site (25).

12. The kit according to claim 11, further comprising a ligating agent, preferably a ligase, capable of ligating together said 3' end (12) of a left ligation oligonucleotide (10) and said 5' end (21) of a right ligation oligonucleotide (20) while hybridized to a target DNA sequence (1) to form a ligated product (30) comprising two UMIs (31, 32) and hybridized to said target DNA sequence (1);

13. The kit according to claim 11 or 12, further comprising a solid phase (4) having preference for DNA sequences of a length in terms of number of deoxyribonucleotides equal to or larger than a minimum length.

14. The kit according to any of the claims 11 to 13, wherein
said first common sequence (43) is one of a P5 sequence (5'-AATGATACGGCGACCACCGA-3', SEQ ID NO: 1) and a P7 sequence (5'-CAAGCAGAAGACGGCATACGAGAT-3', SEQ ID NO: 2); and
said second common sequence (53) is the other of said P5 sequence and said P7 sequence.

15. The kit according to any of the claims 11 to 14, wherein
said sequencing read-1 primer (80) has the nucleotide sequence of ACACTCTTTCCCTACACGACGCTCTTCCGATCT (SEQ ID NO: 3); and
said sequencing read-2 primer (81) has the nucleotide sequence of CTGGAGCTGTCTGCGACTTT (SEQ ID NO: 4), wherein optionally at least one nucleotide is a locked nucleic acid (LNA) nucleotide.

## Patentansprüche

1. Verfahren zum Quantifizieren von Ziel-Desoxyribonukleinsäure (DNA)-Sequenzen (1), wobei das Verfahren die Schritte umfasst:
Kontaktieren (S1) von Ziel-DNA-Sequenzen (1) in jedem Behälter von *N* separaten Behältern, wobei *N* eine ganze Zahl gleich oder größer als zwei ist, unter Hybridisierungsbedingungen mit *M_{L}* linken Ligationsoligonukleotiden (10), die von einem 5'-Ende (11) zu einem 3'-Ende (12) eine Sequenzier-Lese-1-Primerstelle (15), einen jeweiligen ersten eindeutigen molekularen Identifikator (UMI) (14) und eine jeweilige Sequenz (13), die komplementär zu einem jeweiligen ersten Segment (2) einer Ziel-DNA-Sequenz (1) ist, umfassen, und *M_{R}* rechten Ligationsoligonukleotiden (20), die von einem 5'-Ende (21) zu einem 3'-Ende (22) eine jeweilige Sequenz (23), die komplementär zu einem jeweiligen zweiten Segment (3) einer Ziel-DNA-Sequenz (1) ist, einen jeweiligen zweiten UMI (24) und eine Sequenzier-Lese-2-Primerstelle (25) umfassen, wobei *M_{L}* und *M_{R}* jeweils eine ganze Zahl gleich oder größer als zwei sind, wobei der jeweilige erste UMI (14) für jedes der *M_{L}* linken Ligationsoligonukleotide (21) unterschiedlich ist und der jeweilige zweite UMI (24) für jedes der *M_{R}* rechten Ligationsoligonukleotide (20) unterschiedlich ist;
Hinzugeben (S2), in jeden Behälter der *N* separaten Behälter, eines Ligationsmittels, das in der Lage ist, das 3'-Ende (12) eines linken Ligationsoligonukleotids (10) und das 5'-Ende (21) eines rechten Ligationsoligonukleotids (20) miteinander zu ligieren, während sie an eine Ziel-DNA-Sequenz (1) hybridisiert werden, um ein ligiertes Produkt (30) zu bilden, das zwei UMIs (31, 32) umfasst und an die Ziel-DNA-Sequenz (1) hybridisiert ist;
Immobilisieren (S3) des ligierten Produkts (30) in jedem Behälter der *N* separaten Behälter im Komplex mit der Ziel-DNA-Sequenz (1) auf einer festen Phase (4), die eine Präferenz für DNA-Sequenzen mit einer Länge in Bezug auf die Anzahl der Desoxyribonukleotide hat, die gleich oder größer als eine Mindestlänge ist, und Entfernen eines Überstands;
Amplifizieren (S4) des ligierten Produkts (30) in jedem Behälter der *N* separaten Behälter in Gegenwart eines linken Amplifikationsprimers (40), der von einem 5'-Ende (41) zu einem 3'-Ende (42) eine erste gemeinsame Sequenz (43) und die Sequenzier-Lese-1-Primerstelle (44) umfasst, und eines rechten Amplifikationsprimers (50), der von einem 5'-Ende (51) zu einem 3'-Ende (52) eine zweite gemeinsame Sequenz (53), eine Barcode-Sequenz (55) und eine Ligationsoligonukleotid-Bindungsstelle (54), die komplementär zu der Sequenzierungs-Lese-2-Primerstelle (25) ist, umfasst, um ein amplifiziertes Produkt (60) zu bilden, das zwei UMIs (65A, 65B) und eine Barcode-Sequenz (68) umfasst;
Zusammenmischen (S5) des Inhalts der N getrennten Behälter;
Sequenzieren (S6) mindestens eines jeweiligen Teils der amplifizierten Produkte (60) durch Zugabe eines Sequenzier-Lese-1-Primers (80), der die Sequenzier-Lese-1-Primerstelle (15) umfasst, und eines Sequenzier-Lese-2-Primers (81), der die Lese-2-Primerstelle (25) umfasst, um jeweilige Sequenzlesungen zu bilden, die mindestens Nukleotidsequenzen von zwei UMIs (65A, 65B), eine Barcode-Sequenz (68) und eine Ziel-DNA-Sequenz (66) umfassen;
Demultiplexen (S7) der Sequenzlesungen basierend auf Nukleotidsequenzen der Barcode-Sequenzen (68);
Abbilden (S8) der demultiplexierten Sequenzlesungen auf bekannte DNA-Sequenzen basierend auf Nukleotidsequenzen der Ziel-DNA-Sequenz (66); und
Quantifizieren (S9) eindeutiger Ziel-DNA-Sequenzen (1) in den *N* Behältern basierend auf demultiplexierten und abgebildeten Sequenzlesungen und basierend auf Nukleotidsequenzen der UMIs.

2. Verfahren nach Anspruch 1, wobei das Hinzugeben (S2) des Ligationsmittels das Hinzugeben (S2) einer Ligase in jeden Behälter der *N* Behälter umfasst, um das 3'-Ende (12) des linken Ligationsoligonukleotids (10) und das 5'-Ende (21) des rechten Ligationsoligonukleotids (20) miteinander zu ligieren, während sie an die Ziel-DNA-Sequenz (1) hybridisiert werden, um das ligierte Produkt (30) zu bilden.

3. Verfahren nach Anspruch 1 oder 2, wobei das Immobilisieren (S3) des ligierten Produkts (30) das Immobilisieren (S3), in jedem Behälter der *N* Behälter, des an die Ziel-DNA-Sequenz (1) hybridisierten ligierten Produkts (30) auf Magnetkügelchen (4), die eine Präferenz für DNA-Sequenzen mit einer Länge in Bezug auf die Anzahl der Basenpaare gleich oder größer als 100 Basenpaare haben, umfasst.

4. Verfahren nach einem der Ansprüche 1 bis 3, wobei das Amplifizieren (S4) des ligierten Produkts (30) das Durchführen, in jedem Behälter der N Behälter (S4), von 2 bis 30 Zyklen, bevorzugt 10 bis 20 Zyklen der Amplifikation des ligierten Produkts (30) in Gegenwart des linken Amplifikationsprimers (40) und des rechten Amplifikationsprimers (50) umfasst, um das amplifizierte Produkt (60) zu bilden.

5. Verfahren nach einem der Ansprüche 1 bis 4, ferner umfassend das Durchführen (S10) einer größenbasierten Trennung der gemischten amplifizierten Produkte (60) von jedem bei der Amplifikation erzeugten Nebenprodukt (70).

6. Verfahren nach Anspruch 5, wobei das Durchführen (S10) der größenbasierten Trennung umfasst:
Kontaktieren (S11) des gemischten amplifizierten Produkts (60) mit Magnetkügelchen, die eine Präferenz für doppelsträngige DNA-Sequenzen eines Ziellängenintervalls haben, wobei eine Länge des amplifizierten Produkts (60) in das Ziellängenintervall fällt;
Trennen (S12) der Magnetkügelchen von nicht gebundenen Nukleotidsequenzen (70); und
Wiedergewinnen (S13) des amplifizierten Produkts (60) von den magnetischen Kügelchen.

7. Verfahren nach einem der Ansprüche 1 bis 6, wobei das Sequenzieren (S6) des mindestens einen Teils des amplifizierten Produkts (60) das In-situ-Sequenzieren (S6) des mindestens einen Teils des auf einem festen Träger immobilisierten amplifizierten Produkts (60) basierend auf der ersten gemeinsamen Sequenz (63) und/oder der zweiten gemeinsamen Sequenz (69) unter Verwendung des Lese-1-Primers (80) und des Lese-2-Primers (81) umfasst.

8. Verfahren nach einem der Ansprüche 1 bis 7, wobei das Demultiplexen (S7) der Sequenzlesungen das Aufteilen der Sequenzlesungen in Gruppen mit einer gleichen Nukleotidsequenz der Barcode-Sequenzen (68) umfasst, wobei höchstens *n* Fehlpaarungen für Nukleotidsequenzen von Barcode-Sequenzen (68) in einer gleichen Gruppe erlaubt sind, wobei n null oder eins ist.

9. Verfahren nach einem der Ansprüche 1 bis 8, wobei das Abbilden (S8) der demultiplexierten Sequenzlesungen das Aufteilen der demultiplexierten Sequenzlesungen in Gruppen mit einer gleichen Nukleotidsequenz der Ziel-DNA-Sequenz umfasst, wobei höchstens *m* Fehlpaarungen für Nukleotidsequenzen von Ziel-DNA-Sequenzen in einer gleichen Gruppe erlaubt sind, wobei *m* eine ganze Zahl ist, die gleich oder größer als 0, aber nicht größer als zehn, bevorzugt nicht größer als fünf ist.

10. Verfahren nach einem der Ansprüche 1 bis 9, wobei das Quantifizieren (S9) eindeutiger Ziel-DNA-Sequenzen das Quantifizieren (S9) eindeutiger Ziel-DNA-Sequenzen basierend auf dem Bestimmen einer Anzahl von Ziel-DNA-Sequenzen in den N Behältern umfasst, die eine Nukleotidsequenz mit höchstens m Fehlpaarungen aufweisen, eine Nukleotidsequenz der Barcode-Sequenz mit höchstens *n* Fehlpaarungen aufweisen und unterschiedliche Kombinationen des ersten UMI und des zweiten UMI aufweisen.

11. Kit zum Quantifizieren von Ziel-Desoxyribonukleinsäure (DNA)-Sequenzen (1), wobei das Kit umfasst:
*M_{L}* linke Ligationsoligonukleotide (10), die von einem 5'-Ende (11) zu einem 3'-Ende (12) eine Sequenzier-Lese-1-Primerstelle (15), einen jeweiligen ersten eindeutigen molekularen Identifikator (UMI) (14) und eine jeweilige Sequenz (13), die komplementär zu einem jeweiligen ersten Segment (2) einer Ziel-DNA-Sequenz (1) ist, umfassen;
*M_{R}* rechte Ligationsoligonukleotide (20), die von einem 5'-Ende (21) zu einem 3'-Ende (22) eine jeweilige Sequenz (23), die komplementär zu einem jeweiligen zweiten Segment (3) einer Ziel-DNA-Sequenz (1) ist, einen jeweiligen zweiten UMI (24) und eine Sequenzier-Lese-2-Primerstelle (25) umfassen, wobei *M_{L}* und *M_{R}* jeweils eine ganze Zahl gleich oder größer als zwei sind, wobei der jeweilige erste UMI (14) für jedes der *M_{L}* linken Ligationsoligonukleotide (21) unterschiedlich ist und der jeweilige zweite UMI (24) für jedes der *M_{R}* rechten Ligationsoligonukleotide (20) unterschiedlich ist;
einen linken Amplifikationsprimer (40), der von einem 5'-Ende (41) zu einem 3'-Ende (42) eine erste gemeinsame Sequenz (43) und die Sequenzier-Lese-1-Primerstelle (44) umfasst;
einen rechten Amplifikationsprimer (50), der von einem 5'-Ende (51) zu einem 3'-Ende (52) eine zweite gemeinsame Sequenz (53), eine Barcode-Sequenz (55) und eine Ligationsoligonukleotid-Bindungsstelle (54), die komplementär zu der Sequenzier-Lese-2-Primerstelle (25) ist, umfasst;
einen Sequenzier-Lese-1-Primer (80), der die Sequenzier-Lese-1-Primerstelle (15) umfasst; und
einen Sequenzier-Lese-2-Primer (81), der die Lese-2-Primerstelle (25) umfasst.

12. Kit nach Anspruch 11, ferner umfassend ein Ligationsmittel, bevorzugt eine Ligase, die in der Lage ist, das 3'-Ende (12) eines linken Ligationsoligonukleotids (10) und das 5'-Ende (21) eines rechten Ligationsoligonukleotids (20) miteinander zu ligieren, während sie an eine Ziel-DNA-Sequenz (1) hybridisiert werden, um ein ligiertes Produkt (30) zu bilden, das zwei UMIs (31, 32) umfasst und an die Ziel-DNA-Sequenz (1) hybridisiert ist.

13. Kit nach Anspruch 11 oder 12, ferner umfassend eine feste Phase (4), die eine Präferenz für DNA-Sequenzen mit einer Länge in Bezug auf die Anzahl der Desoxyribonukleotide, die gleich oder größer als eine Mindestlänge ist, aufweist.

14. Kit nach einem der Ansprüche 11 bis 13, wobei
die erste gemeinsame Sequenz (43) eine von einer P5-Sequenz (5'-AATGATACGGCGACCACCGA-3', SEQ ID NO: 1) und einer P7-Sequenz (5'-CAAGCAGAAGACGGCATACGAGAT-3', SEQ ID NO: 2) ist; und
die zweite gemeinsame Sequenz (53) die andere von der P5-Sequenz und der P7-Sequenz ist.

15. Kit nach einem der Ansprüche 11 bis 14, wobei der Sequenzier-Lese-1-Primer (80) die Nukleotidsequenz ACACTCTTTCCCTACACGACGCTCTTCCGATCT (SEQ ID NO: 3) aufweist; und
der Sequenzier-Lese-2-Primer (81) die Nukleotidsequenz CTGGAGCTGTCTGCGACTTT (SEQ ID NO: 4) aufweist, wobei optional mindestens ein Nukleotid ein Nukleotid einer verbrückten Nukleinsäure (LNA) ist.

## Revendications

1. Procédé de quantification de séquences d'acide désoxyribonucléique (ADN) cibles (1), ledit procédé comprenant les étapes consistant à :
mettre en contact (S1), dans chaque récipient de N récipients séparés, N étant un nombre entier égal ou supérieur à deux, des séquences d'ADN cibles (1), dans des conditions d'hybridation, avec *M_{L}* oligonucléotides de ligature gauche (10) comprenant, à partir d'une extrémité 5' (11) à une extrémité 3' (12), un site d'amorce de lecture 1 de séquençage (15), un premier identifiant moléculaire unique (UMI) (14) respectif et une séquence (13) respective complémentaire d'un premier segment (2) respectif d'une séquence d'ADN cible (1) et *M_{R}* oligonucléotides de ligature droits (20) comprenant, à partir d'une extrémité 5' (21) à une extrémité 3' (22), une séquence (23) respective complémentaire d'un second segment (3) respectif d'une séquence d'ADN cible (1), un second UMI (24) respectif et un site d'amorce de lecture 2 de séquençage (25), dans lequel *M_{L}* et *M_{R}* sont chacun un nombre entier égal ou supérieur à deux, ledit premier UMI (14) respectif étant différent pour chacun desdits *M_{L}* oligonucléotides de ligature gauche (21) et ledit second UMI (24) respectif étant différent pour chacun desdits *M_{R}* oligonucléotides de ligature droits (20);
ajouter (S2), dans chaque récipient desdits N récipients séparés, un agent de ligature capable de ligaturer ensemble ladite extrémité 3' (12) d'un oligonucléotide de ligature gauche (10) et ladite extrémité 5' (21) d'un oligonucléotide de ligature droit (20) tout en étant hybridé à une séquence d'ADN cible (1) pour former un produit ligaturé (30) comprenant deux UMI (31, 32) et hybridé à ladite séquence d'ADN cible (1) ;
immobiliser (S3), dans chaque récipient desdits N récipients séparés, ledit produit ligaturé (30) en complexe avec ladite séquence d'ADN cible (1) sur une phase solide (4) ayant une préférence pour des séquences d'ADN d'une longueur en termes de nombre de désoxyribonucléotides égale ou supérieure à une longueur minimale et éliminer un surnageant ;
amplifier (S4), dans chaque récipient desdits N récipients séparés, ledit produit ligaturé (30) en présence d'une amorce d'amplification gauche (40) comprenant, à partir d'une extrémité 5' (41) à une extrémité 3' (42), une première séquence commune (43) et ledit site d'amorce de lecture 1 de séquençage (44) et une amorce d'amplification droite (50) comprenant, à partir d'une extrémité 5' (51) à une extrémité 3' (52), une seconde séquence commune (53), une séquence de code-barres (55) et un site de liaison d'oligonucléotide de ligature (54) complémentaire audit site d'amorce de lecture 2 de séquençage (25) pour former un produit amplifié (60) comprenant deux UMI (65A, 65B) et une séquence de code-barres (68) ;
mélanger (S5) ensemble le contenu desdits N récipients séparés ;
séquencer (S6) au moins une partie respective desdits produits amplifiés (60) par addition d'une amorce de lecture 1 de séquençage (80) comprenant ledit site d'amorce de lecture 1 de séquençage (15) et une amorce de lecture 2 de séquençage (81) comprenant ledit site d'amorce de lecture 2 (25) pour former des lectures de séquence respectives comprenant au moins des séquences nucléotidiques de deux UMI (65A, 65B), une séquence de code-barres (68) et une séquence d'ADN cible (66) ;
démultiplexer (S7) lesdites lectures de séquence sur la base des séquences nucléotidiques desdites séquences de code-barres (68) ;
cartographier (S8) lesdites lectures de séquence démultiplexées sur des séquences d'ADN connues sur la base de séquences nucléotidiques de ladite séquence d'ADN cible (66) ; et
quantifier (S9) des séquences d'ADN cible (1) uniques dans lesdits *N* récipients sur la base desdites lectures de séquence démultiplexées et cartographiées et sur la base de séquences nucléotidiques desdites UMI.

2. Procédé selon la revendication 1, dans lequel ajouter (S2) ledit agent de ligature comprend ajouter (S2), dans chaque récipient desdits *N* récipients, une ligase pour ligaturer ensemble ladite extrémité 3' (12) dudit oligonucléotide de ligature gauche (10) et ladite extrémité 5' (21) dudit oligonucléotide de ligature droit (20) tout en étant hybridé à ladite séquence d'ADN cible (1) pour former ledit produit ligaturé (30).

3. Procédé selon l'une des revendications 1 ou 2, dans lequel immobiliser (S3) ledit produit ligaturé (30) comprend immobiliser (S3), dans chaque récipient desdits N récipients, ledit produit ligaturé (30) hybridé à ladite séquence d'ADN cible (1) sur des billes magnétiques (4) ayant une préférence pour des séquences d'ADN d'une longueur en termes de nombre de paires de bases égale ou supérieure à 100 paires de bases.

4. Procédé selon l'une quelconque des revendications 1 à 3, dans lequel amplifier (S4) ledit produit ligaturé (30) comprend effectuer (S4), dans chaque récipient desdits *N* récipients, de 2 à 30 cycles, de préférence de 10 à 20 cycles d'amplification dudit produit ligaturé (30) en présence de ladite amorce d'amplification gauche (40) et de ladite amorce d'amplification droite (50) pour former ledit produit amplifié (60).

5. Procédé selon l'une quelconque des revendications 1 à 4, comprenant en outre effectuer (S10) une séparation sur la base de la taille desdits produits amplifiés mixtes (60) à partir de tout produit secondaire (70) produit dans ladite amplification.

6. Procédé selon la revendication 5, dans lequel effectuer (S10) ladite séparation sur la base de la taille comprend :
mettre en contact (S11) ledit produit amplifié mixte (60) avec des billes magnétiques ayant une préférence pour des séquences d'ADN double brin d'un intervalle de longueur cible, dans lequel une longueur dudit produit amplifié (60) tombe dans ledit intervalle de longueur cible ;
séparer (S12) lesdites billes magnétiques des séquences nucléotidiques non liées (70) ; et
récupérer (S13) ledit produit amplifié (60) à partir desdites billes magnétiques.

7. Procédé selon l'une quelconque des revendications 1 à 6, dans lequel séquencer (S6) ladite au moins une partie dudit produit amplifié (60) comprend séquencer *in situ* (S6) ladite au moins une partie dudit produit amplifié (60) immobilisé sur un support solide sur la base de ladite première séquence commune (63) et/ou ladite seconde séquence commune (69) à l'aide de l'amorce de lecture 1 (80) et de ladite amorce de lecture 2 (81).

8. Procédé selon l'une quelconque des revendications 1 à 7, dans lequel démultiplexer (S7) lesdites lectures de séquence comprend diviser lesdites lectures de séquence en groupes ayant une même séquence nucléotidique que lesdites séquences de code-barres (68) avec au plus *n* mésappariements autorisés pour les séquences nucléotidiques de séquences de code-barres (68) dans un même groupe, *n* étant zéro ou un.

9. Procédé selon l'une quelconque des revendications 1 à 8, dans lequel cartographier (S8) lesdites lectures de séquence démultiplexée comprend diviser les lectures de séquence démultiplexée en groupes ayant une même séquence nucléotidique que ladite séquence d'ADN cible avec au plus *m* mésappariements autorisés pour les séquences nucléotidiques des séquences d'ADN cibles dans un même groupe, *m* étant un nombre entier égal ou supérieur à 0 mais pas plus grand que dix, de préférence pas plus grand que cinq.

10. Procédé selon l'une quelconque des revendications 1 à 9, dans lequel quantifier (S9) des séquences d'ADN cibles uniques comprend quantifier (S9) des séquences d'ADN cibles uniques sur la base de la détermination d'un nombre de séquences d'ADN cibles dans lesdits *N* récipients ayant une séquence nucléotidique avec au plus *m* mésappariements, ayant une séquence nucléotidique de ladite séquence de code-barres avec au plus *n* mésappariements et ayant différentes combinaisons dudit premier UMI et dudit second UMI.

11. Kit pour quantifier les séquences d'acide désoxyribonucléique (ADN) cibles (1), ledit kit comprend :
*M_{L}* oligonucléotides de ligature gauche (10) comprenant, à partir d'une extrémité 5' (11) à une extrémité 3' (12), un site d'amorce de lecture 1 de séquençage (15), un premier identifiant moléculaire unique (UMI) (14) respectif et une séquence (13) respective complémentaire d'un premier segment (2) respectif d'une séquence d'ADN cible (1);
*M_{R}* oligonucléotides de ligature droits (20) comprenant, à partir d'une extrémité 5' (21) à une extrémité 3' (22), une séquence (23) respective complémentaire d'un second segment (3) respectif d'une séquence d'ADN cible (1), un second UMI (24) respectif et un site d'amorce de lecture 2 de séquençage (25), dans lequel *M_{L}* et *M_{R}* sont chacun un nombre entier égal ou supérieur à deux, ledit premier UMI (14) respectif étant différent pour chacun desdits *M_{L}* oligonucléotides de ligature gauche (21) et ledit second UMI (24) respectif étant différents pour chacun desdits *M_{R}* oligonucléotides de ligature droits (20) ;
une amorce d'amplification gauche (40) comprenant, à partir d'une extrémité 5' (41) à une extrémité 3' (42), une première séquence commune (43) et ledit site d'amorce de lecture 1 de séquençage (44) ;
une amorce d'amplification droite (50) comprenant, à partir d'une extrémité 5' (51) à une extrémité 3' (52), une seconde séquence commune (53), une séquence de code-barres (55) et un site de liaison d'oligonucléotide de ligature (54) complémentaire audit site d'amorce de lecture 2 de séquençage (25) ;
une amorce de lecture 1 de séquençage (80) comprenant ledit site d'amorce de lecture 1 de séquençage (15) ; et
une amorce de lecture 2 de séquençage (81) comprenant ledit site d'amorce de lecture 2 (25).

12. Kit selon la revendication 11, comprenant en outre un agent de ligature, de préférence une ligase, capable de ligaturer ensemble ladite extrémité 3' (12) d'un oligonucléotide de ligature gauche (10) et ladite extrémité 5' (21) d'un oligonucléotide de ligature droit (20) tout en étant hybridé à une séquence d'ADN cible (1) pour former un produit ligaturé (30) comprenant deux UMI (31, 32) et hybridé à ladite séquence d'ADN cible (1).

13. Kit selon l'une des revendications 11 ou 12, comprenant en outre une phase solide (4) ayant de préférence pour les séquences d'ADN d'une longueur en termes de nombre de désoxyribonucléotides égale ou supérieure à une longueur minimale.

14. Kit selon l'une quelconque des revendications 11 à 13, dans lequel
ladite première séquence commune (43) est l'une d'une séquence P5 (5'-AATGATACGGCGACCACCGA-3', SEQ ID NO : 1) et d'une séquence P7 (5' -CAAGCAGAAGACGGCATACGAGAT-3', SEQ ID NO : 2) ; et
ladite seconde séquence commune (53) est l'autre de ladite séquence P5 et de ladite séquence P7.

15. Kit selon l'une quelconque des revendications 11 à 14, dans lequel
ladite amorce de lecture 1 de séquençage (80) a la séquence nucléotidique de ACACTCTTTCCCTACACGACGCTCTTCCGATCT (SEQ ID NO : 3) ; et
ladite amorce de lecture 2 de séquençage (81) a la séquence nucléotidique de CTGGAGCTGTCTGCGACTTT (SEQ ID NO : 4), dans lequel facultativement au moins un nucléotide est un nucléotide d'acide nucléique verrouillé (LNA).


### SEQUENCE LISTING

<110> Tervisetehnoloogiate Arenduskeskus AS
<120> QUANTIFYING DNA SEQUENCES
<130> P1349PC00
<150> SE 1750003-4
   <151> 2017-01-05
<160> 25
<170> PatentIn version 3.5
<210> 1
   <211> 20
   <212> DNA
   <213> Artificial Sequence
<220>
   <223> Illumina P5 sequence
<400> 1
   aatgatacgg cgaccaccga 20
<210> 2
   <211> 24
   <212> DNA
   <213> Artificial Sequence
<220>
   <223> Illumina P7 sequence
<400> 2
   caagcagaag acggcatacg agat 24
<210> 3
   <211> 33
   <212> DNA
   <213> Artificial Sequence
<220>
   <223> Sequencing primer
<400> 3
   acactctttc cctacacgac gctcttccga tct 33
<210> 4
   <211> 20
   <212> DNA
   <213> Artificial Sequence
<220>
   <223> Sequencing primer
<400> 4
   ctggagctgt ctgcgacttt 20
<210> 5
   <211> 20
   <212> DNA
   <213> Artificial Sequence
<220>
   <223> Universal sequence of left ligation oligonucleotide
<400> 5
   acacgacgct cttccgatct 20
<210> 6
   <211> 30
   <212> DNA
   <213> Artificial Sequence
<220>
   <223> Oligo-T30 primer
<400> 6
   tttttttttt tttttttttt tttttttttt 30
<210> 7
   <211> 67
   <212> DNA
   <213> Artificial Sequence
<220>
   <223> Left amplification primer
<400> 7
   aatgatacgg cgaccaccga gatctacact aacaacactc tttccctaca cgacgctctt 60
   ccgatct 67
<210> 8
   <211> 50
   <212> DNA
   <213> Artificial Sequence
<220>
   <223> Right amplification primer
<400> 8
   caagcagaag acggcatacg agatgatctg aaagtcgcag acagctccag 50
<210> 9
   <211> 50
   <212> DNA
   <213> Artificial Sequence
<220>
   <223> Right amplification primer
<400> 9
   caagcagaag acggcatacg agatgcctaa aaagtcgcag acagctccag 50
<210> 10
   <211> 50
   <212> DNA
   <213> Artificial Sequence
<220>
   <223> Right amplification primer
<400> 10
   caagcagaag acggcatacg agatcgtgat aaagtcgcag acagctccag 50
<210> 11
   <211> 50
   <212> DNA
   <213> Artificial Sequence
<220>
   <223> Right amplification primer
<400> 11
   caagcagaag acggcatacg agattggtca aaagtcgcag acagctccag 50
<210> 12
   <211> 50
   <212> DNA
   <213> Artificial Sequence
<220>
   <223> Right amplification primer
<400> 12
   caagcagaag acggcatacg agatattggc aaagtcgcag acagctccag 50
<210> 13
   <211> 50
   <212> DNA
   <213> Artificial Sequence
<220>
   <223> Right amplification primer
<400> 13
   caagcagaag acggcatacg agatctgatc aaagtcgcag acagctccag 50
<210> 14
   <211> 50
   <212> DNA
   <213> Artificial Sequence
<220>
   <223> Right amplification primer
<400> 14
   caagcagaag acggcatacg agatgtagcc aaagtcgcag acagctccag 50
<210> 15
   <211> 50
   <212> DNA
   <213> Artificial Sequence
<220>
   <223> Right amplification primer
<400> 15
   caagcagaag acggcatacg agattacaag aaagtcgcag acagctccag 50
<210> 16
   <211> 50
   <212> DNA
   <213> Artificial Sequence
<220>
   <223> Right amplification primer
<400> 16
   caagcagaag acggcatacg agatatcagt aaagtcgcag acagctccag 50
<210> 17
   <211> 50
   <212> DNA
   <213> Artificial Sequence
<220>
   <223> Right amplification primer
<400> 17
   caagcagaag acggcatacg agataggaat aaagtcgcag acagctccag 50
<210> 18
   <211> 50
   <212> DNA
   <213> Artificial Sequence
<220>
   <223> Right amplification primer
<400> 18
   caagcagaag acggcatacg agattagttg aaagtcgcag acagctccag 50
<210> 19
   <211> 50
   <212> DNA
   <213> Artificial Sequence
<220>
   <223> Right amplification primer
<400> 19
   caagcagaag acggcatacg agatatcgtg aaagtcgcag acagctccag 50
<210> 20
   <211> 50
   <212> DNA
   <213> Artificial Sequence
<220>
   <223> Right amplification primer
<400> 20
   caagcagaag acggcatacg agattgagtg aaagtcgcag acagctccag 50
<210> 21
   <211> 50
   <212> DNA
   <213> Artificial Sequence
<220>
   <223> Right amplification primer
<400> 21
   caagcagaag acggcatacg agatgccatg aaagtcgcag acagctccag 50
<210> 22
   <211> 50
   <212> DNA
   <213> Artificial Sequence
<220>
   <223> Right amplification primer
<400> 22
   caagcagaag acggcatacg agattgttgg aaagtcgcag acagctccag 50
<210> 23
   <211> 50
   <212> DNA
   <213> Artificial Sequence
<220>
   <223> Right amplification primer
<400> 23
   caagcagaag acggcatacg agatagcatc aaagtcgcag acagctccag 50
<210> 24
   <211> 51
   <212> DNA
   <213> Artificial Sequence
<220>
   <223> Left ligation oligonucleotide
<220>
   <221> misc_feature
   <222> (21)..(24)
   <223> UMI
<220>
   <221> misc_feature
   <222> (25)..(51)
   <223> Sequence complementary to a segment of a target DNA sequence
<400> 24
   acacgacgct cttccgatct nnnnnnnnnn nnnnnnnnnn nnnnnnnnnn n 51
<210> 25
   <211> 51
   <212> DNA
   <213> Artificial Sequence
<220>
   <223> Right ligation oligonucleotide
<220>
   <221> misc_feature
   <222> (1)..(27)
   <223> Sequence complementary to segment of a target DNA sequence
<220>
   <221> misc_feature
   <222> (28)..(31)
   <223> UMI
<400> 25
   nnnnnnnnnn nnnnnnnnnn nnnnnnnnnn nctggagctg tctgcgactt t 51